(19)

**Europäisches Patentamt**
**European Patent Office**
**Office européen des brevets**

(11) **EP 1 952 822 A1**

(12) **EUROPEAN PATENT APPLICATION**

(43) Date of publication:
**06.08.2008 Bulletin 2008/32**

(51) Int Cl.:
*A61K 38/36* *(2006.01)*　*C07K 14/745* *(2006.01)*
*C12N 9/64* *(2006.01)*　*G01N 33/68* *(2006.01)*

(21) Application number: **07101259.5**

(22) Date of filing: **26.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **NOVO NORDISK A/S**
**2880 Bagsvaerd (DK)**

(72) Inventor: **The designation of the inventor has not yet been filed**

(54) **Factor VII polypeptides with increased affinity to platelets**

(57) The invention relates to methods for screening of new coagulation factor VIIa variants with increased affinity to thrombocytes as well as factor VIIa variants identified by this method.

EP 1 952 822 A1

## Description

### FIELD OF THE INVENTION

The present invention relates to new coagulation factor VIIa polypeptides with increased affinity to blood platelets (thrombocytes) as well as methods for the screening of these new coagulation factor VIIa polypeptides.

### BACKGROUND OF THE INVENTION

Haemostasis is a complex physiological process which ultimately results in the arrest of bleeding. This is dependent on the proper function of three main components: blood vessels (especially the endothelial lining), coagulation factors, and platelets. Once a haemostatic plug is formed, the timely activation of the fibrinolytic system is equally important to prevent further unnecessary activation of coagulation. Any malfunction of this system (due to a reduced number, or molecular dysfunction, of the haemostatic components or increased activation of the fibrinolytic components) may lead to clinical bleeding such as, e.g., haemorrhagic diathesis of varying severity.

In most physiological situations, coagulation is triggered by the interaction of circulating activated coagulation factor VII (FVIIa) with tissue factor (TF) subsequent to exposure of TF at the site of an injury. Endogenous FVIIa becomes proteolytically active only after forming a complex with TF. Normally, functional TF is expressed in the deep layers of the vessel wall and is only exposed to blood following injury. This ensures a highly localized activation of coagulation and prevents disseminated coagulation. TF also seems to exist in a non-active form, so-called encrypted TF. The regulation of encrypted versus active TF is still unknown.

Activated recombinant human factor VII (rFVIIa) is indicated for the treatment of bleeding episodes in haemophilia A or B patients with inhibitors to Factor VIII or Factor IX. When given in high (pharmacological) doses, rFVIIa is able to activate factor X on an activated platelet via an interaction with negatively charged phospholipids, independent of tissue factor (TF) and initiate local thrombin generation which is important for the formation of the haemostatic plug.

FVII polypeptides according to the invention may be useful for administration to mammals, particularly humans, to control bleeding disorders, particularly bleeding disorders which are caused by clotting factor deficiencies (haemophilia A and B), or clotting factor inhibitors or bleeding disorders in patients not suffering from haemophilia A or B, for example, in patients suffering from von Willebrand's disease. Patients with von Willebrand's disease have a defective primary haemostasis because they lack or have an abnormal von Willebrand factor protein. Bleeding disorders are also seen in patients with a normally functioning blood coagulation cascade and may be caused by a defective platelet function, thrombocytopati, thrombocytopenia, or even by unknown reasons. Furthermore, FVIIa polypeptides may be used for preventing or treating excessive bleedings in other patients, such as, for example, bleedings in association with tissue damage, for example surgery or trauma. FVIIa polypeptides may be used as well as when the bleeding is diffuse and poorly responding to current haemostatic techniques and therapies (such as, e.g. haemorrhagic gastritis and profuse uterine bleeding). FVIIa polypeptides may also be suitable for the treatment of bleedings occurring in organs with limited possibility for mechanical haemostasis such as brain, inner ear region, and eyes as well as in association with the process of taking biopsies from various organs and in laparoscopic surgery.

### SUMMARY OF THE INVENTION

The present invention describes the identification of a mechanism by which FVII polypeptides binds with increased affinity to blood platelets. The inventors of the present application have identified that a platelet receptor, human platelet glycoprotein Ib alpha, is involved in the binding of recombinant FVIIa to activated platelets at a site of a bleeding. This has made it possible to screen and select FVII polypeptides with increased binding affinity to platelets and thereby increased functional activity.

In a first aspect the present invention relates to a method for the identification of a FVII polypeptide with increased binding affinity to activated human platelets as compared to human wild-type FVIIa, said method comprising the steps of:

a) providing a FVII polypeptide different from human wild-type FVIIa;
b) testing said FVII polypeptide different from human wild-type FVIIa for the binding affinity to human platelet glycoprotein Ib alpha;
c) testing said FVII polypeptide different from human wild-type FVIIa for functional coagulation activity;
d) selecting said FVII polypeptide with both i) functional coagulation activity substantially the same as human wild-type FVIIa; and ii) increased binding affinity of said FVII polypeptide to human platelet glycoprotein Ib alpha as compared to human wild-type FVIIa.

In a second aspect the present invention relates to a FVII polypeptide obtainable by a method for the identification of a FVII polypeptide with increased binding affinity to activated human platelets as compared to human wild-type FVIIa, said method comprising the steps of:

a) providing a FVII polypeptide different from human wild-type FVIIa;
b) testing said FVII polypeptide different from human wild-type FVIIa for the binding affinity to human platelet glycoprotein Ib alpha;
c) testing said FVII polypeptide different from human wild-type FVIIa for functional coagulation activity;
d) selecting said FVII polypeptide with both i) functional coagulation activity substantially the same as human wild-type FVIIa; and ii) increased binding affinity of said FVII polypeptide to human platelet glycoprotein Ib alpha as compared to human wild-type FVIIa.

The present invention also provides methods for preventing or treating a bleeding episode which are carried out by administering to a patient in need of such treatment an effective amount for such treatment of a FVII polypeptide according to the invention.

## DETAILED DESCRIPTION OF THE INVENTION

The inventors of the present invention have provided evidence for a receptor for FVIIa on platelets. Their results point towards an interaction of rFVIIa with platelet glycoprotein Ib alpha (GPIba) on activated platelets. The binding of rFVIIa to GPIba is facilitated by phosphatidylserine on the outer surface of activated platelets. Concerning efficacy and safety it is highly attractive to generate a FVIIa variant with high affinity to GPIba and thereby to the activated platelet. A FVIIa variant with improved binding properties to activated platelets could have improve haemostatic potential. This FVIIa compound with higher efficacy that is restricted to activated platelets would improve safety.

Thus in a broad aspect the present invention relates to FVII polypeptides with increased affinity to activated thrombocytes as compared to wild type human rFVIIa.

A FVII polypeptide with optimal interaction with GPIba may bind stronger to activated platelets. This molecule could comprise a FVII polypeptide with altered glycan structure or with amino acid substitution in e.g. one of the EGF-like domains of FVII.

In one aspect the present invention relates to a method for the identification of a FVII polypeptide with increased binding affinity to activated human platelets as compared to human wild-type FVIIa, said method comprising the steps of:

a) providing a FVII polypeptide different from human wild-type FVIIa;
b) testing said FVII polypeptide different from human wild-type FVIIa for the binding affinity to human platelet glycoprotein Ib alpha;
c) testing said FVII polypeptide different from human wild-type FVIIa for functional coagulation activity;
d) selecting said FVII polypeptide with both i) functional coagulation activity substantially the same as human wild-type FVIIa; and ii) increased binding affinity of said FVII polypeptide to human platelet glycoprotein Ib alpha as compared to human wild-type FVIIa.

In one embodiment the binding affinity of the FVII polypeptide is measured by any method selected from the list consisting of:

a) binding of said FVII polypeptide to CHO cells transfected with human glycoprotein Ib alpha;
b) binding of said FVII polypeptide to activated platelets, such as in a static platelet adhesion assay, or a flow cytometry assay;
c) binding of said FVII polypeptide to immobilised Glycocalicin, such as in a Biacore or ELISA assay; and
d) binding of Glycocalicin to an immobilized preparation of said FVII polypeptide, such as in a Biacore or ELISA assay.

In one embodiment, the functional coagulation activity of the FVII polypeptide is measured by any assay selected from

a) in vitro hydrolysis assay;
b) in vitro proteolysis assay;
c) FX activation on activated platelets and
d) thrombin generation assay on activated platelets

In one embodiment the affinity of the FVII polypeptide according to the invention is 10 % higher than that of human wild-type FVIIa, such as 20 % higher than that of human wild-type FVIIa, such as 30 % higher than that of human wild-type FVIIa, such as 40 % higher than that of human wild-type FVIIa. such as 50 % higher than that of human wild-type FVIIa such as 100 % higher than that of human wild-type FVIIa.

In one embodiment the FVII polypeptide according to the invention is provided by random mutagenesis, site-directed mutagenesis, or modification of glycostructure.

In one embodiment the FVII polypeptide according to the invention comprises one or more substitutions relative to the amino acid sequence of SEQ ID NO: 1 in any one of the EGF-like domain 1 or EGF-like domain 2 of said FVIIa polypeptide.

In one embodiment the substitution relative to the amino acid sequence of SEQ ID NO: 1 in any one of the EGF-like domain 1 or EGF-like domain 2 of the FVIIa polypeptide according to the invention is in one or more position selected from the list consisting of Lys38, Ile42, Gly47, Asp48, Ala51, Ser53, Gln56, Gly58, Gln66, Ser67, Leu73, Pro74, Ala75, His84, Asp86, Asp87, Leu89, Glu94, Gly97, Asp104, His105, Thr106, Gly107, Thr108, Lys109, Ser111, Arg113, Glu116, Ser119, Leu121, Ala122, Asp123, Gly124, Thr128, Pro129, Thr130, Val131, Glu132, and Glu142.

In one embodiment the Factor VII polypeptide according to the invention has a modified structure of N-linked and/or O-linked oligosaccharides.

The term "subject" as used herein is intended to mean any animal, in particular mammals, such as humans, and may, where appropriate, be used interchangeably with the term "patient".

The term "binding affinity" as used herein is intended to mean the strength of interaction between FVII polypeptides and GPIb-V-IX complex or the extracellular part of GPIba, also called glycocalicin. An important parameter that characterizes such noncovalent interaction at equilibrium is the dissociation constant ($K_d$). At equilibrium, the Kd is the product of the free receptor concentration and the free ligand concentration divided with the concentration of the receptor-ligand-complex given by the following expression:

$$Kd = [receptor_{free}] \times [ligand_{free}] / ([receptor\text{-}ligand_{complex}]).$$

A low Kd value means a high binding affinity and is related to a strong interaction between FVII polypeptides and GPIba. Whereas, a relative higher Kd value means a relative lower binding affinity and is thus related to a weaker interaction between FVII polypeptides and GPIba. In surface plasmon resonance analysis the Kd is defined as the ratio between K(off) rate and K(on) rate (the rate of dissociation of the ligand and the rate of the association of the ligand, respectively) as described previously (Lenting PJ et al. J Biol Chem 274: 23734 - 23739, 1999). In other assays, such as the static platelet adhesion assay a Kd value can not be determined and the binding affinity of a given FVII polypeptide to GPIba is evaluated relative to wild-type rFVIIa and thus reflect a semiquantitative evaluation.

The phrase "FVII polypeptide different from human wild-type FVIIa" as used herein is intended to mean a FVII polypeptide different in any physical aspect from recombinant Factor VII with wild-type sequence (i.e. the amino acid sequence disclosed in U.S. Patent No. 4,784,950) expressed by transfected CHO cells grown at serum-containing conditions and purified as described in (Jurlander B et al. Thromb Hemost 27: 373-383, 2001), i.e. Novoseven®. The phrase "FVII polypeptide different from human wild-type FVIIa" is therefore intended to encompass both Factor VIIa polypeptides with wild-type amino acid sequence grown under different conditions to give a FVII polypeptide, that is different by e.g. glycosylation patterns, as well as amino acid variants of Factor VII, Factor VII derivatives and Factor VII conjugates.

The terms "human platelet glycoprotein Ib alpha" and "GPIba" as used herein refers to the polypeptide having the amino acid sequence disclosed in reference (Wenger RH et al. Biochem Biophys Res Commun: 156(1), 389-95, 1988). GPIba is part of the GPIb-V-IX receptor complex where two molecules of GPIba is associated with two GPIb beta molecules, two GPIX molecules and one GPV molecule (Berndt M C Thromb Haemost 86: 178-88, 2001; Andrews RK et al. Int J Biochem Cell Biol 35: 1170-1174, 2003). GPIb-V-IX is found on platelets where it functions as receptor for von Willebrand factor (vWF) and other proteins. Initial platelet adhesion to the subendothelium at high shear is mediated via vWF binding. Other platelet GPIb-IX-V mediated cell-interactions involve binding to leucocytes via the counter receptor Mac-1 and binding to endothelium via P-selectin expressed on activated endothelial cells. Furthermore, GPIba binds a-thrombin thereby functioning as a co-factor for thrombin-mediated PAR-1 activation. Both Factor XII and High Molecular Weight Kininogen bind GPIba and down-regulate thrombin-induced platelet aggregation. GPIba has been detected on endothelial cells (Wu G et al. Blood 90: 2660-2669, 1997) where it is upregulated by tumour necrosis factor a and may be involved in endothelial cell migration on vWF (Lian J et al. Exp Cell Res 252: 114-22, 1999).

When used herein, the term "Factor VII polypeptide" encompasses wild-type Factor VII (i.e. a polypeptide having the amino acid sequence disclosed in U.S. Patent No. 4,784,950), as well as variants of Factor VII, Factor VII derivatives, Factor VII conjugates, and Glycoform variants of FVIIa exhibiting substantially the same or improved biological activity relative to wild-type Factor VII. The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. The term "Factor VII polypeptide" also encompasses polypeptides, including variants, in which the Factor VIIa biological activity has been substantially modified or somewhat reduced relative to the activity of wild-type Factor VIIa.

The biological activity of Factor VIIa in blood coagulation derives from its ability to (i) bind to TF and (ii) catalyze the proteolytic cleavage of Factor IX or Factor X to produce activated Factor IX or X (Factor IXa or Xa, respectively).

In the present context, the term "functionally active Factor VII polypeptide" refers to "Factor VII polypeptide" that have been proteolytically processed to yield their respective bioactive forms, and which frequently are referred to as "Factor VIIa polypeptides".

In some important embodiments, the Factor VII polypeptide is functionally active.

As used herein, "wild type human FVIIa" is a polypeptide having the amino acid sequence disclosed in U.S. Patent No. 4,784,950.

The term "Factor VII derivative" as used herein, is intended to designate a FVII polypeptide exhibiting substantially the same or improved biological activity relative to wild-type Factor VII, in which one or more of the amino acids of the parent peptide have been genetically and/or chemically and/or enzymatically modified, e.g. by alkylation, glycosylation, PEGylation, acylation, ester formation or amide formation or the like. This includes but is not limited to PEGylated human Factor VIIa, cysteine-PEGylated human Factor VIIa and variants thereof. Non-limiting examples of Factor VII derivatives includes GlycoPegylated FVII derivatives as disclosed in WO 03/31464 and US Patent applications US 20040043446, US 20040063911, US 20040142856, US 20040137557, and US 20040132640 (Neose Technologies, Inc.); FVII conjugates as disclosed in WO 01/04287, US patent application 20030165996, WO 01/58935, WO 03/93465 (Maxygen ApS) and WO 02/02764, US patent application 20030211094 (University of Minnesota).

The term "improved biological activity" refers to FVII polypeptides with i) substantially the same or increased proteolytic activity compared to recombinant wild type human Factor VIIa or ii) to FVII polypeptides with substantially the same or increased TF binding activity compared to recombinant wild type human Factor VIIa or iii) to FVII polypeptides with substantially the same or increased half life in blood plasma compared to recombinant wild type human Factor VIIa. The term "PEGylated human Factor VIIa" means human Factor VIIa, having a PEG molecule conjugated to a human Factor VIIa polypeptide. It is to be understood, that the PEG molecule may be attached to any part of the Factor VIIa polypeptide including any amino acid residue or carbohydrate moiety of the Factor VIIa polypeptide. The term "cysteine-PEGylated human Factor VIIa" means Factor VIIa having a PEG molecule conjugated to a sulfhydryl group of a cysteine introduced in human Factor VIIa.

Non-limiting examples of additional substitutions of amino acids in the Factor VII polypeptide providing Factor VII polypeptides with substantially the same or increased proteolytic activity compared to recombinant wild type human Factor VIIa include S52A, S60A (Lino et al., Arch. Biochem. Biophys. 352: 182-192, 1998); substitutions providing FVII polypeptides exhibiting increased proteolytic stability as disclosed in U.S. Patent No. 5,580,560; substitutions providing FVII polypeptides that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); substitutions in FVII polypeptides as disclosed in WO 02/077218; and substitutions in FVII polypeptides, which exhibit increased proteolytic stability as disclosed in WO 02/38162 (Scripps Research Institute); substitutions in FVII polypeptides, which provides polypeptides having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and substitutions in FVII polypeptides as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of further substitutions in FVII polypeptides providing FVII polypeptides having increased biological activity compared to wild-type FVIIa include substitutions as disclosed in WO 01/83725, WO 02/22776, WO 02/077218, WO 03/027147, WO 04/029090, WO 03/027147; WO 02/38162 (Scripps Research Institute); and FVII polypeptides with enhanced activity as disclosed in JP 2001061479 (Chemo-Sero-Therapeutic Res Inst.).

Examples of further substitutions in the FVII polypeptides of the present invention include, without limitation, L305V, L305V/M306D/D309S, L305I, L305T, F374P, V158T/M298Q, V158D/E296V/M298Q, K337A, M298Q, V158D/M298Q, L305V/K337A, V158D/E296V/M298Q/L305V, V158D/E296V/M298Q/K337A, V158D/E296V/M298Q/L305V/K337A, K157A, E296V, E296V/M298Q, V158D/E296V, V158D/M298K, and S336G, L305V/K337A, L305V/V158D, L305V/E296V, L305V/M298Q, L305V/V158T, L305V/K337A/V158T, L305V/K337A/M298Q, L305V/K337A/E296V, L305V/K337A/V158D, L305V/V158D/M298Q, L305V/V158D/E296V, L305V/V158T/M298Q, L305V/V158T/E296V, L305V/E296V/M298Q, L305V/V158D/E296V/M298Q, L305V/V158T/E296V/M298Q, L305V/V158T/K337A/M298Q, L305V/V158T/E296V/K337A, L305V/V158D/K337A/M298Q, L305V/V158D/E296V/K337A, L305V/V158D/E296V/M298Q/K337A, L305V/V158T/E296V/M298Q/K337A, S314E/K316H, S314E/K316Q, S314E/L305V, S314E/K337A, S314E/V158D, S314E/E296V, S314E/M298Q, S314E/V158T, K316H/L305V, K316H/K337A, K316H/V158D, K316H/E296V, K316H/M298Q, K316H/V158T, K316Q/L305V, K316Q/K337A, K316Q/V158D, K316Q/E296V, K316Q/M298Q, K316Q/V158T, S314E/L305V/K337A, S314E/L305V/V158D, S314E/L305V/E296V, S314E/L305V/M298Q, S314E/L305V/V158T, S314E/L305V/K337A/V158T, S314E/L305V/K337A/M298Q, S314E/L305V/K337A/E296V, S314E/L305V/K337A/V158D, S314E/L305V/V158D/M298Q, S314E/L305V/V158D/E296V, S314E/L305V/V158T/M298Q, S314E/L305V/V158T/E296V, S314E/L305V/E296V/M298Q, S314E/L305V/V158D/E296V/M298Q, S314E/L305V/V158T/E296V/M298Q, S314E/L305V/V158T/K337A/M298Q, S314E/L305V/V158T/E296V/K337A, S314E/L305V/V158D/K337A/M298Q, S314E/L305V/V158D/E296V/K337A, S314E/L305V/V158D/E296V/M298Q/K337A, S314E/L305V/V158T/E296V/M298Q/K337A, K316H/L305V/K337A, K316H/L305V/V158D, K316H/L305V/E296V, K316H/L305V/M298Q, K316H/L305V/V158T, K316H/L305V/K337A/V158T, K316H/L305V/K337A/M298Q, K316H/L305V/K337A/E296V, K316H/L305V/K337A/V158D, K316H/L305V/V158D/M298Q, K316H/L305V/V158D/E296V, K316H/L305V/V158T/M298Q, K316H/L305V/VI58T/E296V, K316H/L305V/E296V/M298Q, K316H/L305V/V158D/E296V/M298Q, K316H/L305V/VI58T/E296V/M298Q, K316H/L305V/V158T/K337A/M298Q, K316H/L305V/VI58T/E296V/K337A, K316H/L305V/V158D/K337A/M298Q, K316H/L305V/V158D/E296V/K337A, K316H/L305V/VI58D/E296V/M298Q/K337A, K316H/L305V/VI58T/E296V/M298Q/K337A, K316Q/L305V/K337A, K316Q/L305V/V158D, K316Q/L305V/E296V, K316Q/L305V/M298Q, K316Q/L305V/V158T, K316Q/L305V/K337A/V158T, K316Q/L305V/K337A/M 298Q, K316Q/L305V/K337A/E296V, K316Q/L305V/K337A/VI58D, K316Q/L305V/VI58D/M298Q, K316Q/L305V/VI58D/E296V, K316Q/L305V/V158T/M298Q, K316Q/L305V/V158T/E296V, K316Q/L305V/E296V/M298Q, K316Q/L305V/VI58D/E296V/M298Q, K316Q/L305V/V158T/E296V/M298Q, K316Q/L305V/V158T/K337A/M298Q, K316Q/L305V/V158T/E296V/K337A, K316Q/L305V/V158D/K337A/M298Q, K316Q/L305V/V158D/E296V/K337A, K316Q/L305V/V158D/E296V/M298Q/K337A, K316Q/L305V/V158T/E296V/M298Q/K337A, F374Y/K337A, F374Y/V158D, F374Y/E296V, F374Y/M298Q, F374Y/V158T, F374Y/S314E, F374Y/L305V, F374Y/L305V/K337A, F374Y/L305V/V158D, F374Y/L305V/E296V, F374Y/L305V/M298Q, F374Y/L305V/V158T, F374Y/L305V/S314E, F374Y/K337A/S314E, F374Y/K337A/V158T, F374Y/K337A/M298Q, F374Y/K337A/E296V, F374Y/K337A/V158D, F374Y/V158D/S314E, F374Y/V158D/M298Q, F374Y/V158D/E296V, F374Y/V158T/S314E, F374Y/V158T/M298Q, F374Y/V158T/E296V, F374Y/E296V/S314E, F374Y/S314E/M298Q, F374Y/E296V/M298Q, F374Y/L305V/K337A/V158D, F374Y/L305V/K337A/E296V, F374Y/L305V/K337A/M298Q, F374Y/L305V/K337A/V158T, F374Y/L305V/K337A/S314E, F374Y/L305V/V158D/E296V, F374Y/L305V/V158D/M298Q, F374Y/L305V/V158D/S314E, F374Y/L305V/E296V/M298Q, F374Y/L305V/E296V/V158T, F374Y/L305V/E296V/S314E, F374Y/L305V/M298Q/V158T, F374Y/L305V/M298Q/S314E, F374Y/L305V/V158T/S314E, F374Y/K337A/S314E/V158T, F374Y/K337A/S314E/M298Q, F374Y/K337A/S314E/E296V, F374Y/K337A/S314E/V158D, F374Y/K337A/V158T/M298Q, F374Y/K337A/V158T/E296V, F374Y/K337A/M298Q/E296V, F374Y/K337A/M298Q/V158D, F374Y/K337A/E296V/V158D, F374Y/V158D/S314E/M298Q, F374Y/V158D/S314E/E296V, F374Y/V158D/M298Q/E296V, F374Y/V158T/S314E/E296V, F374Y/V158T/5314E/M298Q, F374Y/V158T/M298Q/E296V, F374Y/E296V/S314E/M298Q, F374Y/L305V/M298Q/K337A/S314E, F374Y/L305V/E296V/K337A/S314E, F374Y/E296V/M298Q/K337A/S314E, F374Y/L305V/E296V/M298Q/K337A, F374Y/L305V/E296V/M298Q/S314E, F374Y/V158D/E296V/M298Q/K337A, F374Y/V158D/E296V/M298Q/S314E, F374Y/L305V/V158D/K337A/S314E, F374Y/V158D/M298Q/K337A/S314E, F374Y/V158D/E296V/K337A/S314E, F374Y/L305V/V158D/E296V/M298Q, F374Y/L305V/V158D/M298Q/K337A, F374Y/L305V/V158D/E296V/K337A, F374Y/L305V/V158D/M298Q/S314E, F374Y/L305V/V158D/E296V/S314E, F374Y/V158T/E296V/M298Q/K337A, F374Y/V158T/E296V/M298Q/S314E, F374Y/L305V/V158T/K337A/S314E, F374Y/VI58T/M298Q/K337A/S314E, F374Y/V158T/E296V/K337A/S314E, F374Y/L305V/V158T/E296V/M298Q, F374Y/L305V/V158T/M298Q/K337A, F374Y/L305VIV158T/E296V/K337A, F374Y/L305V/V158T/M298Q/S314E, F374Y/L305V/VI58T/E296V/S314E, F374Y/E296V/M298Q/K337A/V158T/S314E, F374Y/V158D/E296V/M298Q/K337A/S314E, F374Y/L305V/V158D/E296V/M298Q/S314E, F374Y/L305V/E296V/M298Q/V158T/S314E, F374Y/L305V/E296V/M298Q/K337A/V158T, F374Y/L305V/E296V/K337A/V158T/S314E, F374Y/L305V/M298Q/K337A/V158T/S314E, F374Y/L305V/VI58D/E296V/M298Q/K337A, F374Y/L305V/V158D/E296V/K337A/S314E, F374Y/L305V/V158D/M298Q/K337A/S314E, F374Y/L305V/E296V/M298Q/K337A/V158T/S314E, F374Y/L305V/V158D/E296V/M298Q/K337A/S314E, S52A, S60A; R152E, S344A, T106N, K143N/N145T, V253N, R290N/A292T, G291N, R315N/V317T, K143N/N145T/R315N/V317T; and substitutions, additions or deletions in the amino acid sequence from 233Thr to 240Asn; substitutions, additions or deletions in the amino acid sequence from 304Arg to 329Cys; and substitutions, additions or deletions in the amino acid sequence from 153Ile to 223Arg.

The terms "variant" or "variants", as used herein, is intended to designate Factor VII having the amino acid sequence disclosed in U.S. Patent No. 4,784,950, wherein one or more amino acids of the parent protein have been substituted by another amino acid and/or wherein one or more amino acids of the parent protein have been deleted and/or wherein one or more amino acids have been inserted in protein and/or wherein one or more amino acids have been added to the parent protein. Such addition can take place either at the N-terminal end or at the C-terminal end of the parent protein or both. The "variant" or "variants" within this definition still have FVII activity in its activated form. In one embodiment a variant is 70 % identical with the amino acid sequence disclosed in U.S. Patent No. 4,784,950. In one embodiment a variant is 80 % identical with the amino acid sequence disclosed in U.S. Patent No. 4,784,950. In another embodiment a variant is 90 % identical with the amino acid sequence disclosed in U.S. Patent No. 4,784,950. In a further embodiment a variant is 95 % identical with the amino acid sequence disclosed in U.S. Patent No. 4,784,950.

The term "EGF-like domain 1" as used herein means the amino acid sequence 46-82 of SEQ ID NO:1. The term "EGF-like domain 2" as used herein means the amino acid sequence 87-128 of SEQ ID NO:1.

FVIIa N-glycoform variants:

In one embodiment of the invention, the FVII polypeptide different from human wild-type FVIIa exhibits one or more of the following glycoform patterns, i.e. a glycoform FVIIa variant, wherein:

(i) Between about 94-100% of the oligosaccharide chains contain at least one sialic acid residue, such as, e.g., between about 94-99%, between about 95-98%, or between about 96-97%. In different embodiments, at least about 94%, 95%, 96%, 97%, 98, or 99% of the oligosaccharide chains contain at least one sialic acid residue.

(ii) 6% or less of the oligosaccharide chains are neutral, such as, e.g., between about 0.5-6% or between 1.5-6% or between about 2-4% or between 0.5-4% or between 0.5-2%.

(iii) Less than about 16%, preferably less than about 10% of the oligosaccharide chains contain at least one terminal galactose, such as, e.g., between about 6-10% or between about 8-9%;

(iv) Less than about 25%, preferably less than about 10% of the oligosaccharide chains contain at least one terminal GalNAc residue, such as, e.g., between about 6-9% or between about 7-8%;

(v) Less than about 30, preferably less than about 25% of the oligosaccharide chains contain at least one uncapped antenna, such as, e.g., between about 11-23% or between about 12-18%; and

(vi) At least about 2%, preferably at least about 5%, more preferably at least about 10% or 20%; and most preferably, at least about 40%, of the oligosaccharide chains contain at least one fucose linked $\alpha$1->3 to an antennary N-acetylglucosamine residue (i.e., an N-acetylglucosamine residue that is linked $\beta$1->2,4, or 6 to a Man residue).

It will be understood that each of (i)-(vi) may represent a distinct glycoform pattern that may encompassed by embodiments of the present invention, i.e., the glycoform pattern of a FVII polypeptide may be described by only one of (i)-(vi). Alternatively, the glycoform pattern of a FVII polypeptide encompassed by the invention may be described by more than one of (i)-(vi).

Furthermore, a FVII polypeptide encompassed by the invention may be described by one or more of (i)-(vi) in combination with one or more other structural features. For example, the invention encompasses FVII polypeptides in which the sialic acid residues (Neu5Ac or Neu5Gc) are linked to galactose exclusively in an $\alpha$2->3 configuration. The invention may also encompass FVII polypeptides that contain fucose linked $\alpha$ 1->6 to a core N-acetylglucosamine and/or fucose linked $\alpha$1->3 to an antennary N-acetylglucosamine. The FVII polypeptides of the invention may encompass a FVII polypeptide in which more than 99% of the oligosaccharide chains contain at least one sialic acid residue *and* (a) the sialic acid residues are linked exclusively in an $\alpha$2->3 configuration and/or (b) there are fucose residues linked to core N-acetylglucosamines and/or (c) a detectable number of antenna terminate in N-acetylgalactosamine. The FVII polypeptides of the invention may encompass a FVII polypeptide in which more than 99% of the oligosaccharide chains contain at least one sialic acid residue and the sialic acid residues are linked to galactose exclusively in an $\alpha$2->3 configuration. The FVII polypeptides of the invention may encompass a FVII polypeptide comprising wild-type Factor VIIa in which more than 99% of the oligosaccharide chains contain at least one sialic acid residue and at least some of the oligosaccharide chains comprise N-acetylgalactosamine.

The pattern of N-linked and/or O-linked oligosaccharides may be determined using any method known in the art, including, without limitation: high-performance liquid chromatography (HPLC); capillary electrophoresis (CE); nuclear magnetic resonance (NMR); mass spectrometry (MS) using ionization techniques such as fast-atom bombardment, electrospray, or matrix-assisted laser desorption (MALDI); gas chromatography (GC); and treatment with exoglycosidases in conjunction with anion-exchange (AIE)-HPLC, size-exclusion chromatography (SEC), mass spectroscopy (MS), gel electrophoresis (SDS-PAGE, CE-PAGE), isoelectric focusing gels, or iso-electric focusing capillary electrophoresis (CE-IEF) See, e.g., Weber et al., Anal. Biochem. 225:135 (1995); Klausen et al., J. Chromatog. 718:195 (1995); Morris et al., in Mass Spectrometry of Biological Materials, McEwen et al., eds., Marcel Dekker, (1990), pp 137-167; Conboy et al., Biol. Mass Spectrom. 21:397, 1992; Hellerqvist, Meth. Enzymol, 193:554 (1990); Sutton et al., Anal. Biohcem. 318:34 (1994); Harvey et al., Organic Mass Spectrometry 29:752 (1994).

FVIIa O-glycoform variants as well as enzymes used in the preparation of these FVIIa O-glycoform variants may be prepared as described in WO 2005/111225.

Preparations and formulations:

Human purified Factor VIIa suitable for use in the present invention is preferably made by DNA recombinant technology, e.g. as described by Hagen et al., Proc.Natl.Acad.Sci. USA 83: 2412-2416, 1986 or as described in European Patent No. 200.421 (ZymoGenetics).

The Factor VII variants described herein may be produced by means of recombinant nucleic acid techniques. In general, a cloned wild-type Factor VII nucleic acid sequence is modified to encode the desired protein. This modified sequence is then inserted into an expression vector, which is in turn transformed or transfected into host cells. Higher eukaryotic cells, in particular cultured mammalian cells, are preferred as host cells. The complete nucleotide and amino acid sequences for human Factor VII are known (see U.S. 4,784,950, where the cloning and expression of recombinant human Factor VII is described). The bovine Factor VII sequence is described in Takeya et al., J. Biol. Chem. 263: 14868-14872, 1988. It will be understood that any conventional technique may be used to produce Factor VIIa sequence variants.

Factor VII may also be produced by the methods described by Broze and Majerus, J. Biol.Chem. 255 (4): 1242-1247, 1980 and Hedner and Kisiel, J. Clin.Invest. 71: 1836-1841, 1983. These methods yield Factor VII without detectable amounts of other blood coagulation Factors. An even further purified Factor VII preparation may be obtained by including an additional gel filtration as the final purification step.

Chemical and/or enzymatic modification of wild-type Factor VIIa or Factor VIIa sequence variants may be achieved by any means known in the art. Suitable modifications include, without limitation, chemical glycan modifications as described in EP application, EP06120000 (Novo Nordisk A/S), C-terminal modifications as described in WO2006013202, glycosyltransferase mediated modification as described in WO2006035057, dendrimer modification as described in WO2005014049, carboxypeptidase mediated terminal modification (as described, e.g., in WO2005035553-A2, WO9520039-A, and WO9838285-A), transglutaminase-mediated modification (as described, e.g., WO2005070468), autocatalytic/endopeptidase-mediated transpeptidation (as described, e.g., in WO2006013202-A2); thiol-mediated modification (as described, e.g., in WO2002077218 and PCT/EP/2006063310) and amine-mediated modification (as described, e.g., in WO02/02764.

Factor VII and Factor VII-related polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853, 1972; Thomas, U.S. Patent No. 4,456,591; and Hedner et al., J. Clin. Invest. 71: 1836,1983. Alternatively, Factor VII may be activated by passing it through an ion-exchange chromatography column, such as Mono Q® (Pharmacia) or the like (Thim L et al. Biochemistry 27: 7785-7793, 1988; Jurlander B et al. Thromb Hemost 27: 373-383, 2001). The resulting activated Factor VII may then be formulated and administered as described below.

The present invention encompasses therapeutic administration of Factor VII polypeptides, which is achieved using formulations that comprise Factor VIIa preparations. As used herein, a "Factor VII preparation" refers to a plurality of Factor VIIa polypeptides, including variants and chemically modified forms, that have been separated from the cell in which they were synthesized, whether a cell of origin or a recombinant cell that has been programmed to synthesize the Factor VII polypeptide according to the invention.

Separation of polypeptides from their cell of origin may be achieved by any method known in the art, including, without limitation, removal of cell culture medium containing the desired product from an adherent cell culture; centrifugation or filtration to remove non-adherent cells; and the like.

Optionally, Factor VII polypeptides may be further purified. Purification may be achieved using any method known in the art, including, without limitation, affinity chromatography, such as, e.g., on an anti-Factor VII antibody column (see, e.g., Wakabayashi et al., J. Biol. Chem. 261:11097, 1986; and Thim et al., Biochem. 27:7785, 1988); hydrophobic interaction chromatography; ion-exchange chromatography; size exclusion chromatography; electrophoretic procedures (e.g., preparative isoelectric focusing (IEF), differential solubility (e.g., ammonium sulfate precipitation), or extraction and the like. See, generally, Scopes, Protein Purification, Springer-Verlag, New York, 1982; and Protein Purification, J.-C. Janson and Lars Ryden, editors, VCH Publishers, New York, 1989. Following purification, the preparation preferably contains less than about 10% by weight, more preferably less than about 5% and most preferably less than about 1%, of non-Factor VII proteins derived from the host cell.

Factor VII and Factor VII-related polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853, 1972; Thomas, U.S. Patent No. 4,456,591; and Hedner et al., J. Clin. Invest. 71:1836, 1983. Alternatively, Factor VII may be activated by passing it through an ion-exchange chromatography column, such as Mono Q® (Pharmacia) or the like. The resulting activated Factor VII may then be formulated and administered as described below.

Pharmaceutical compositions or formulations for use in the present invention comprise a Factor VIIa preparation in combination with, preferably dissolved in, a pharmaceutically acceptable carrier, preferably an aqueous carrier or diluent. A variety of aqueous carriers may be used, such as water, buffered water, 0.4% saline, 0.3% glycine and the like. The preparations of the invention can also be formulated into liposome preparations for delivery or targeting to the sites of injury. Liposome preparations are generally described in, e.g., U.S. Patents Nos. 4,837,028, 4,501,728, and 4,975,282. The compositions may be sterilised by conventional, well-known sterilisation techniques. The resulting aqueous solutions may be packaged for use or filtered under aseptic conditions and lyophilised, the lyophilised preparation being combined with a sterile aqueous solution prior to administration.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

Treatment regimen:

In practicing the present invention, Factor VII polypeptide according to the invention may be administered to a subject as a single dose comprising a single-dose-effective amount for treating or preventing the bleeding episodes in a subject with bleedings, or in a staged series of doses which together comprise an effective amount for treating the bleeding episodes. An effective amount of Factor VII polypeptide according to the invention (see below) refers to the amount of Factor VII polypeptide which, when administered in a single dose or in the aggregate of multiple doses, or as part of any other type of defined treatment regimen, produces a measurable improvement in at least one clinical parameter associated with bleedings. When Factor VIIa polypeptides with different activity are administered, an effective amount may be determined by comparing the coagulant activity of the new Factor VIIa polypeptides with that of known Factor VIIa polypeptides and adjusting the amount to be administered proportionately to the predetermined effective dose for the known Factor VIIa polypeptides,

Administration of Factor VII polypeptide according to the invention according to the present invention is preferably initiated within about 6 hours after occurrence of the bleeding episode, such as, e.g., within about 4 hours, within about 2 hours, or within about 1 hour. Alternatively, administration may be initiated at any time before start of surgery in subjects in need of such surgery, such as within about 6 hours before surgery, such as, e.g., within about 4 hours, within about 2 hours, or within about 1 hour before surgery e.g., immediately before surgery.

Administration of a single dose refers to administration of an entire dose of Factor VII polypeptide according to the invention as a bolus over a period of less than about 5 minutes. In some embodiments, the administration occurs over a period of less than about 2.5 minutes, and, in some, over less than about 1 min. Typically, a single-dose effective amount comprises at least about 10 $\mu$g/kg human Factor VII polypeptide according to the invention, such as, at least about 20 $\mu$g/kg, 40 $\mu$g/kg, 50 $\mu$g/kg, 75 $\mu$g/kg, or 90 $\mu$g/kg, or at least 150 $\mu$g/kg Factor VIIa.

In some embodiments, following administration of a single dose of Factor VII polypeptide according to the invention, the subject receives no further Factor VII polypeptide according to the invention for an interval of at least about 30 minutes. In some embodiments the post-administration interval is at least about 45 minutes, such as at least about 1 hour, at least about 1.5 hours, or at least about 2 hours.

In other embodiments, the subject receives Factor VII polypeptide according to the invention according to the following regimen: (i) The subject receives a first amount of Factor VII polypeptide according to the invention comprising at least about 10 $\mu$g/kg; (ii) after a period of at least about 30 minutes, a second amount of Factor VII polypeptide according to the invention is administered, the amount comprising at least about 10 $\mu$g/kg; and (iii) after a period of at least about 30 minutes from administration of the second dose, a third amount of Factor VII polypeptide according to the invention is administered, the amount comprising at least about 10 $\mu$g/kg. After a period of at least about 30 minutes following the administration of the third amount, the subject may then receive a further (fourth) amount of Factor VII polypeptide according to the invention comprising at least about 10 $\mu$g/kg.

In other embodiments, the first amount of Factor VII polypeptide according to the invention comprises at least about 10 $\mu$g/kg, such as at least about 20 $\mu$g/kg, such as at least about 40 $\mu$g/kg, such as at least about 60 $\mu$g/kg, such as at least about 80 $\mu$g/kg, such as at least about 100 $\mu$g/kg or at least about 150 $\mu$g/kg ; in other embodiments, the second amount of Factor VII polypeptide according to the invention comprises at least about 75 $\mu$g/kg, such as at least about 90 $\mu$g/kg; in other embodiments, the third (and optionally fourth) amount of a Factor VII polypeptide according to the invention comprises at least about 75 $\mu$g/kg, such as at least about 90 $\mu$g/kg,

In one embodiment, the first dose comprises about 200 $\mu$g/kg, the second dose about 100 $\mu$g/kg, and the third (and optionally fourth) dose about 100 $\mu$g/kg.

In other embodiments, the subject receives the second amount of a Factor VII polypeptide according to the invention after a period of at least about 45 minutes from the first administration, such as at least about 1 hour, at least about 1.5 hours, at least about 2 hours, at least about 2.5 hours, or at least about 3 hours.

In other embodiments, the subject receives the third (and optionally fourth) amount of Factor VII polypeptide according to the invention after a period of at least about 45 minutes from the previous administration, such as at least about 1 hour, at least about 1.5 hours, at least about 2 hours, at least about 2.5 hours, or at least about 3 hours.

In one embodiment, the subject receives a first dose comprising about 200 $\mu$g/kg; after a period of about 1 hour, the subject receives a second dose comprising about 100 $\mu$g/kg, and after a period of about 3 hours from the first dose, the subject receives a third dose comprising about 100 $\mu$g/kg.

The following table illustrates different non-limiting embodiments of the invention:

Table 1:

| Time post-injury or post excision | 1st Dose | Time to 2nd dose | 2nd Dose (optional) | Time to 3rd dose | 3rd Dose (optional) | Additional Doses |
|---|---|---|---|---|---|---|
| 0 - ≤1h | >10 mcg/kg | 0-1h | >10mcg/kg | 0-1h | >10mcg/kg | As needed |
| | | 1-2h | | 1-2h | | |
| | | ≥3h | | ≥3h | | |
| | 100 mcg/kg | 0-1h | 50-100 mcg/kg | 0-1h | 50-100 mcg/kg | |
| | | 1-2h | | 1-2h | | |
| | | ≥3 h | | ≥3 h | | |
| | ≥150 mcg/kg | 0-1h | 50-100 mcg/kg | 0-1h | 50-100 mcg/kg | |
| | | 1-2h | | 1-2h | | |
| | | ≥3 h | | ≥3 h | | |
| >1 - ≤3h | > 10 mcg/kg | 0-1h | >10mcg/kg | 0-1h | > 10mcg/kg | As needed |
| | | 1-2h | | 1-2h | | |
| | | ≥3h | | ≥3h | | |
| | 100 mcg/kg | 0-1h | 50-100 mcg/kg | 0-1h | 50-100 mcg/kg | |
| | | 1-2h | | 1-2h | | |
| | | ≥ 3 h | | ≥3 h | | |
| | ≥150 mcg/kg | 0-1h | 50-100 mcg/kg | 0-1h | 50-100 mcg/kg | |
| | | 1-2h | | 1-2h | | |
| | | ≥ 3 h | | ≥ 3 h | | |
| >3 - ≤ 6h | >50mcg/kg | 0-1h | >50mcg/kg | 0-1h | >50mcg/kg | As needed |
| | | 1-2h | | 1-2h | | |
| | | ≥ 3 h | | ≥ 3 h | | |
| | 100 mcg/kg | 0-1h | 50-100 mcg/kg | 0-1h | 50-100 mcg/kg | |
| | | 1-2h | | 1-2h | | |
| | | ≥ 3 h | | ≥ 3 h | | |
| | ≥150 mcg/kg | 0-1h | 50-100 mcg/kg | 0-1h | 50-100 mcg/kg | |
| | | 1-2h | | 1-2h | | |
| | | ≥3 h | | ≥3 h | | |

(continued)

| Time post-injury or post excision | 1st Dose | Time to 2nd dose | 2nd Dose (optional) | Time to 3rd dose | 3rd Dose (optional) | Additional Doses |
|---|---|---|---|---|---|---|
| >6 - ≤ 12h | >50mcg/kg | 0-1h | >50mcg/kg | 0-1h | >50mcg/kg | As needed |
| | | 1-2h | | 1-2h | | |
| | | ≥3 h | | ≥3 h | | |
| | 100 mcg/kg | 0-1h | 50-100 mcg/kg | 0-1h | 50-100 mcg/kg | |
| | | 1-2h | | 1-2h | | |
| | | ≥3 h | | ≥3 h | | |
| | ≥150 mcg/kg | 0-1h | 50-100 mcg/kg | 0-1h | 50-100 mcg/kg | |
| | | 1-2h | | 1-2h | | |
| | | ≥3 h | | ≥3 h | | |

It will be understood that the effective amount of Factor VII polypeptide according to the invention, as well as the overall dosage regimen, may vary according to the subject's haemostatic status, which, in turn, may be reflected in one or more clinical parameters, including, e.g., relative levels of circulating coagulation factors; amount of blood lost; rate of bleeding; hematocrit, and the like. It will be further understood that the effective amount may be determined by those of ordinary skill in the art by routine experimentation, by constructing a matrix of values and testing different points in the matrix.

For example, in one series of embodiments, the invention encompasses (i) administering a first dose of a Factor VII polypeptide according to the invention; (ii) assessing the subject's coagulation status after a predetermined time; and (iii) based on the assessment, administering a further dose of Factor VII polypeptide according to the invention if necessary. Steps (ii) and (iii) may be repeated until satisfactory hemostasis is achieved.

According to the invention, a Factor VII polypeptide according to the invention may be administered by any effective route, including, without limitation, intravenous, intramuscular, subcutaneous, mucosal, and pulmonary routes of administration. Preferably, administration is by an intravenous route.

Combination treatments:

The present invention encompasses combined administration of an additional agent in concert with a Factor VII polypeptide according to the invention. In some embodiments, the additional agent comprises a coagulant, including, without limitation, a coagulation factor such as, e.g. Factor V (see, e.g., PCT/DK02/00736), Factor VIII, Factor IX (see, e.g., WO 02/062376), Factor X, Factor XI, Factor XIII (see, e.g., WO 01/85198), Fibrinogen, thrombin, TAFI (see, e.g., PCT/DK02/00734), Antifibrinolytics such as, e.g., PAI-1, aprotinin, epsilon-aminocaproic acid or tranexamic acid (see, e.g., PCT/DK02/00735; PCT/DK02/00742; PCT/DK02/00751; PCT/DK02/00752);, various antithrombotic treatments, as well as transfusions with platelet, RBC, FFP, oxygen carriers, the various bypassing agents and fluid therapies (colloids/crystalloids) or any combination thereof; inhibitors of tissue factor pathway inhibitor (TFPI inhibitors) (see, e.g., WO 01/85199); protein C inhibitors (see, e.g., PCT/DK02/00737); thrombomodulin (see, e.g., PCT/DK02/00738); protein S inhibitors (see, e.g., PCT/DK02/00739); tissue plasminogen activator inhibitors (see, e.g., PCT/DK02/00740); a2-antiplasmin (see, e.g., PCT/DK02/00741).

It will be understood that, in embodiments comprising administration of combinations of Factor VIIa with other agents, the dosage of Factor VII polypeptide according to the invention may on its own comprise an effective amount and additional agent(s) may further augment the therapeutic benefit to the subject. Alternatively, the combination of Factor VIIa or equivalent and the second agent may together comprise an effective amount for treating the bleeding episode. It will also be understood that effective amounts may be defined in the context of particular treatment regimens, including, e.g., timing and number of administrations, modes of administrations, formulations, etc.

The present invention is further illustrated by the following examples which, however, are not to be construed as limiting the scope of protection. The features disclosed in the foregoing description and in the following examples may, both separately and in any combination thereof, be material for realising the invention in diverse forms thereof.

**BRIEF DESCRIPTION OF THE DRAWINGS**

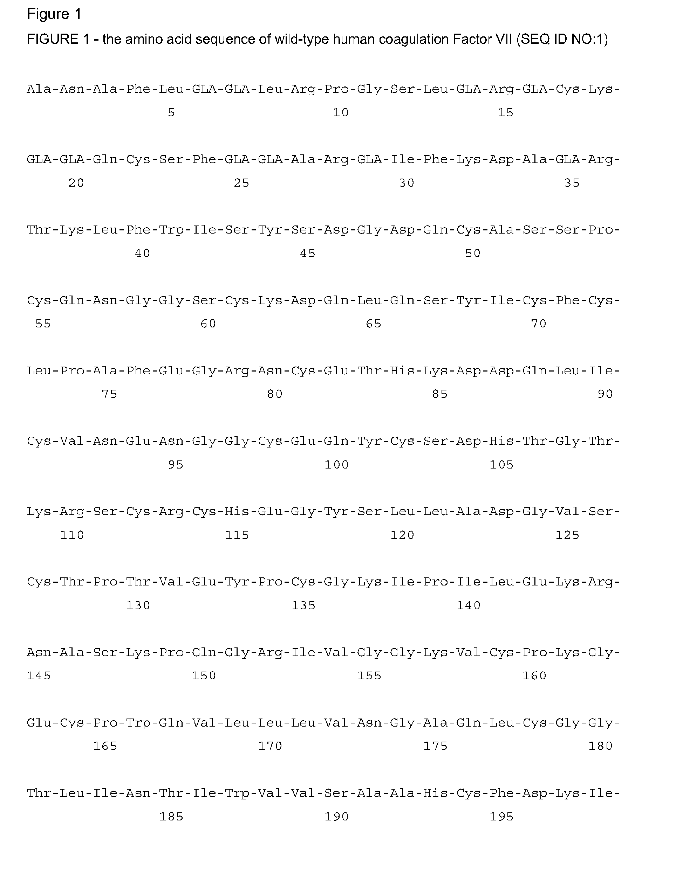

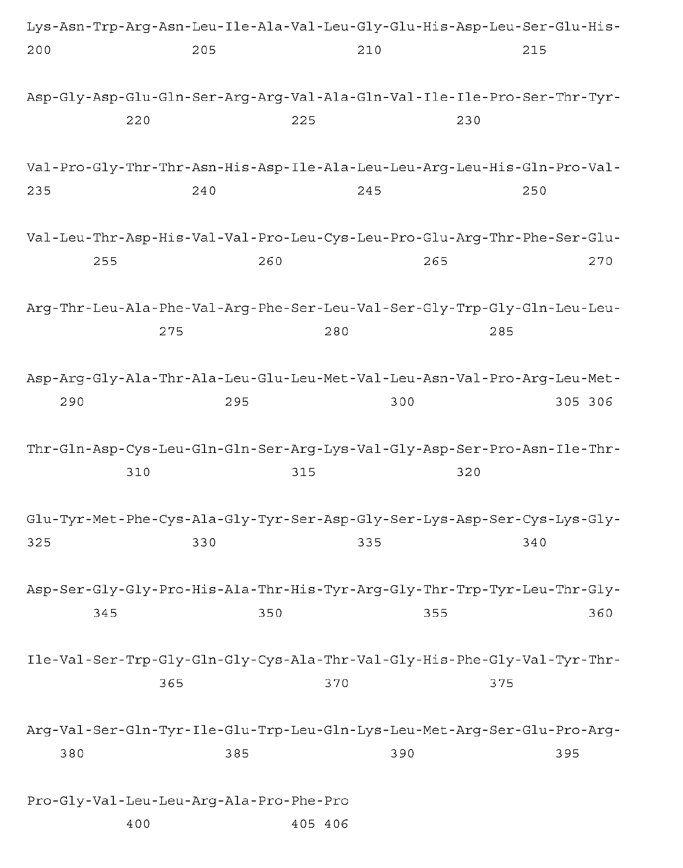

Figure 1: The full amino acid sequence of native (wild type) human coagulation Factor VII (SEQ ID NO:1).

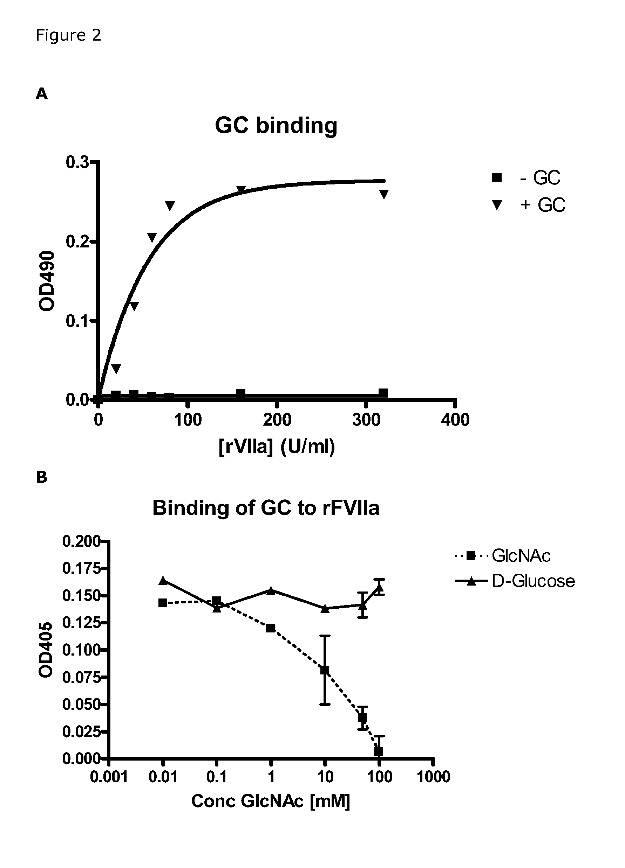

Figure 2: A: The graph shows the binding of purified glycocalicin (GC) to immobilized rFVIIa. Various concentrations of rFVIIa were immobilized on 96-well plates followed by addition of glycocalicin. The amount of bound glycocalicin was determined by ELISA. B: The graph shows the binding of purified glycocalicin to immobilized rFVIIa in the presence of various concentrations of N-acetylglucosamine (GlcNAc) or D-glucose. The amount of bound glycocalicin was determined by ELISA.

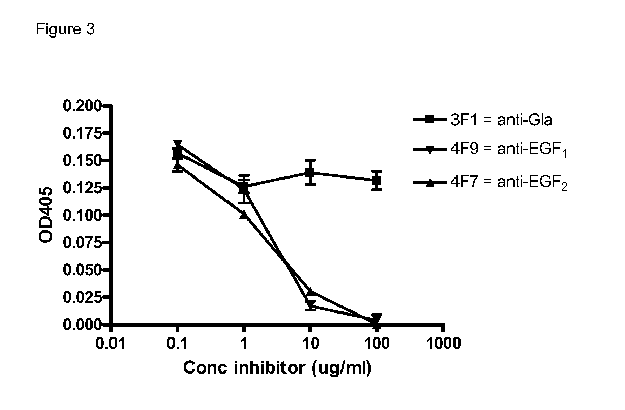

Figure 3: The graph shows the binding of purified glycocalicin to immobilized rFVIIa in the presence of monoclonal antibodies against human rFVIIa-Gla domain (3F1), rFVIIa-EGF1 (4F9) or rFVIIa-EGF2 (4F7) domain. The amount of bound glycocalicin was determined by ELISA.

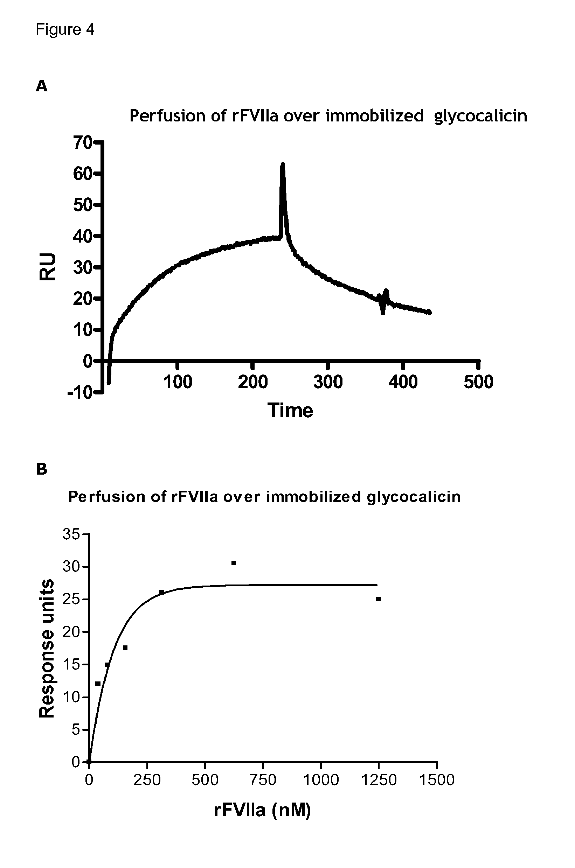

Figure 4: A: The graph shows the binding of rFVIIa to glycocalicin. rFVIIa were perfused over a glycocalicin coated CM5-sensor chip with a flow rate of 5 $\mu$l/min and the amount of bound rFVIIa was measured by BIAcore2000 biosensor system. B: The graph shows the binding of rFVIIa to glycocalicin. Various concentrations of rFVIIa were perfused over a glycocalicin coated CM5-sensor chip with a flow rate of 5 $\mu$l/min and the amount of bound rFVIIa was measured by BIAcore2000 biosensor system. The maximal response was evaluated at equilibrium.

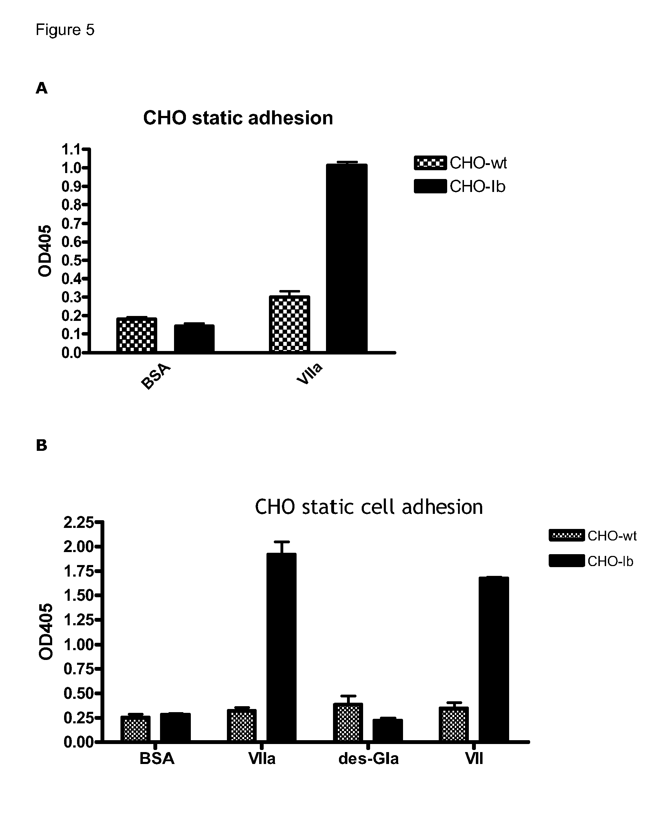

Figure 5, A, B: The columns illustrate the binding of CHO cells expressing GPIba on immobilized rFVIIa. Microtiter plates were coated with rFVIIa (VIIa), zymogen rFVII (VII), rFVIIa without the Gla-domain (des-Gla) or bovine serum albumin (BSA). CHO cells stabil transfected with GPIba (CHO-Ib) or wild type CHO cells (CHO-wt) were added to coated micro titter plates for 90 min at 37°C and the amount of adhered cells was evaluated by optical density at 405 nm.

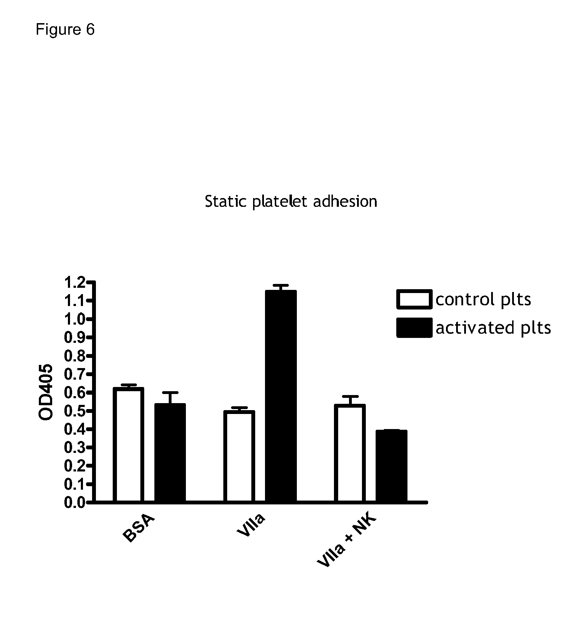

Figure 6: The columns show the binding of platelets to immobilized rFVIIa. Microtiter plates were coated with rFVIIa (VIIa) or bovine serum albumin (BSA) followed by incubation for 90 min at 37°C with untreated platelets (control plts) or activated platelets. Some platelets were preincubated with the GPIba-cleaving metalloproteinase, Naja Kaouthia (NK). The amount of adhered platelets was evaluated by optical density at 405 nm.

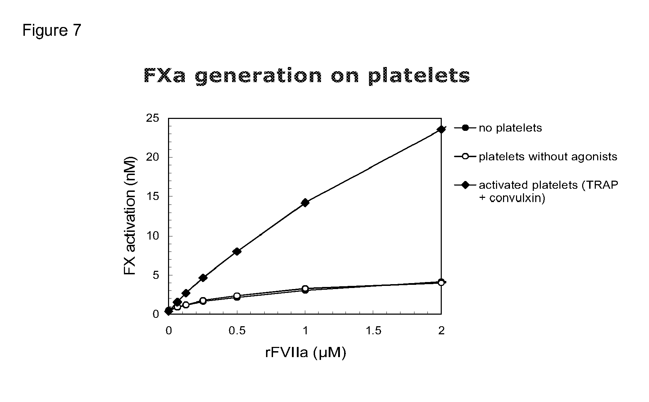

Figure 7: The figure shows the FXa generation on human platelets. Platelets were activated with TRAP combined with convulxin, or left non-stimulated and added to microtiter plates and mixed with various concentrations of rFVIIa and FX in buffer with CaCl$_2$. After incubation for 20 min the FX activation were determined by the use of a FXa chromogenic substrate and the absorbance was measured at 405 nm in a Spectramax plate reader and converted to nM FXa by the use of a standard curve

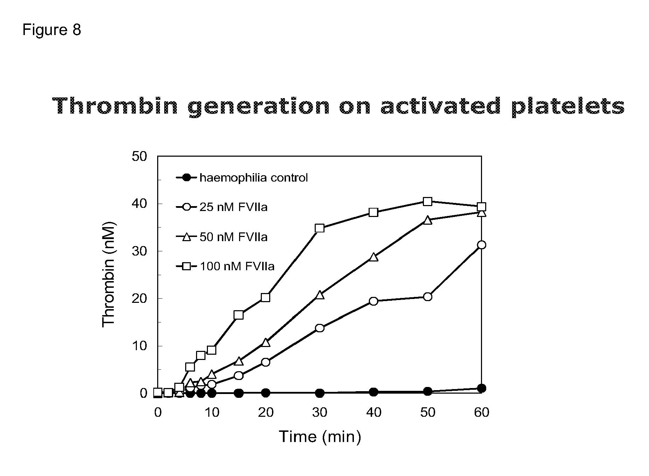

Figure 8: The figure shows the thrombin generation on activated human platelets. Platelets were isolated and activated with the thrombin receptor agonist peptide SFLLRN. Prothrombin, FX, FV, ATIII, TFPI, CaCl$_2$ and rFVIIa were added and the thrombin generation was measure at timed intervals by the use of a chromogenic substrat.The absorbance was measured at 405 nm in a Spectramax plate reader and converted to nM thrombin by the use of a standard curve

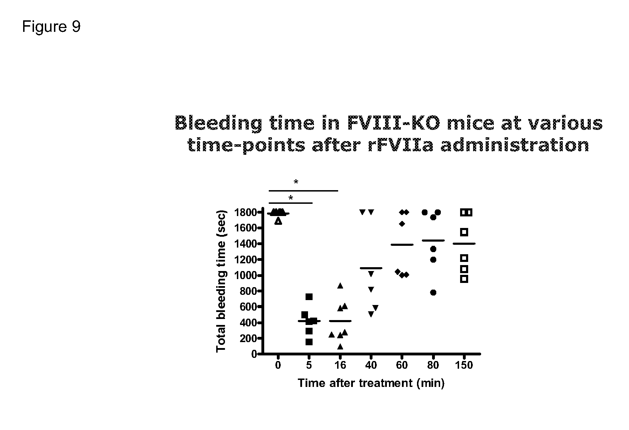

Figure 9: Shows the bleeding time in FVIII-knock out (KO) mice at various time-points after rFVIIa administration. FVIII-KO mice were i.v. administrated and tail clipping performed after 5, 16, 40, 60, 80, and 180 min. Then, the total bleeding time were determined.

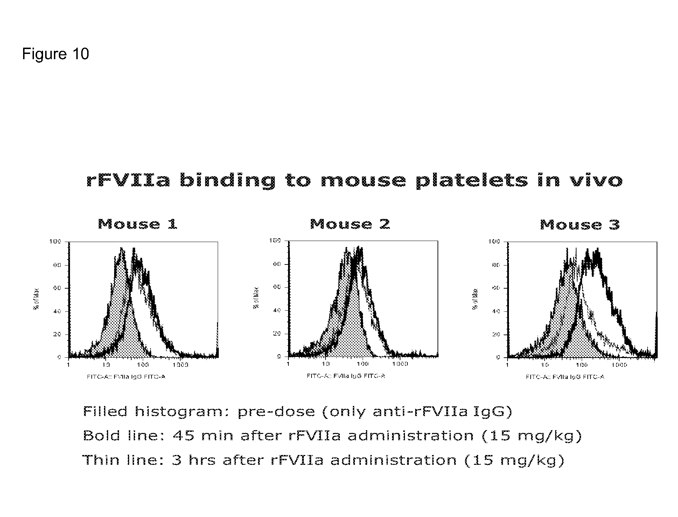

Figure 10: Shows rFVIIa binding to mouse platelets in vivo. rFVIIa was i.v. administrated to normal mice after 45min or 3 hrs eye blood was taken, platelets were isolated and binding of rFVIIa to platelets measured at various time-points by the use of flow cytometry.

**EXAMPLES**

Example 1

rFVIIa variants

The terminology for amino acid substitutions used the following examples is as follows. The first letter represent the amino acid naturally present at a position of SEQ ID NO:1. The following number represent the position in SEQ ID NO:1. The second letter represent the different amino acid substituting for (replacing) the natural amino acid. An example is M298Q, where an methionine at position 298 of SEQ ID NO:1 is replaced by a glutamine. In another example, V158T/M298Q, the valine in position 158 of SEQ ID NO:1 is replaced by a threonine and the methionine in position 298 of SEQ ID NO:1 is replaced by a Glutamine in the same Factor VII polypeptide,

FVII polypeptides according to the invention to be used according to the invention may be prepared according to published international patent applications, e.g. WO 01/83725, WO 02/22776, WO 02/077218, WO 03/027147, WO 04/029090, WO 05/075635, European patent application with application number 05108713.8 (Novo Nordisk A/S), WO 02/38162 and JP 2001061479.

Example 2

Interaction between rFVIIa variants and glycocalicin measured by ELISA

A proteolytic fragment of the majority of the extracellular part of GPIba (glycocalicin, GC) binds to immobilized FVIIa. Glycocalicin was purified from plasma as described (Weeterings C et al, Arterioscler Thromb Vasc Biol 26(3): 670-5, 2006). In short, glycocalicin was purified from fresh-frozen plasma by immunoaffinity chromatography using a mouse monoclonal antibody against human GPIba (prepared at University of Utrecht using standard methods) coupled to CNBr-activated Sepharose 4B (5 mg antibody/ml column)(Pharmacia, Uppsala, Sweden), followed by affinity chromatography on Wheat-germ agglutinin-agarose (Fluka Chemie, Buchs, Switzerland). The purified glycocalicin appeared as a single band on SDS-PAGE and concentration was measured by using a BCA protein assay kit (Pierce, Rockford, IL) using bovine serum albumin as a standard. rFVIIa or rFVIIa-des-Gla (rFVIIa without the Gla-domain) was immobilized for 2 hrs at 37°C on a Costar (2595) 96-wells plate at the indicated concentrations. After blocking the wells with 2% BSA in Tris-buffered saline (TBS, 50 mM Tris, 150 mM NaCl, pH 7.4) for at least 30 min, the wells were incubated with 2 ug/ml glycocalicin for 90 min at room temperature. Bound glycocalicin was detected with a rabbit polyclonal antibody against GPIb (prepared at University of Utrecht using standard methods) and a peroxidase-labeled swine-anti-rabbit antibody, followed by 1,3,5-trinitrobenzene (TNB) staining. Results were obtained by measuring optical density at 490 nm on a SpectraMax Plate Reader. The results showed that rFVIIa (Figure 2A) and rFVIIa-des-Gla (not shown) bind to glycocalicin. Furthermore, the binding was inhibited by excess of N-actetylglucosamine (GlcNac) whereas D-glucose failed to inhibit binding (Figure 2B). This demonstrates that rFVIIa and rFVIIa-des-Gla bind to GPIba, and indicates that GlcNac in either the glycosylated stack of GPIba or in rFVIIa is important for the interaction.

Example 3

The EGF1 and EGF2 domains of rFVIIa are involved in binding to the extracellular part of GPIbα measured by ELISA,

rFVIIa was immobilised on microtitre plates as described in example 2. Then, anti-FVIIa-EGF1 domain antibody (mouse anti-human FVII-4F9, IgG1 subclone A106B5, prepared at Novo Nordisk using standard methods), anti-FVIIa-EGF2 domain antibody (mouse anti-human FVII-4F7, IgG1 subclone A4B101, prepared at Novo Nordisk using standard methods) and anti-FVIIa-Gla antibody (mouse anti-human FVII-3F1 IgG1 subclone A2B1C4, prepared at Novo Nordisk using standard methods) were added together with glycocalicin, and bound glycocalicin was detected as described under example 2. Results showed that the binding of glycocalicin was inhibited by anti-FVIIa-EGF1 and anti-FVII-EGF2 domain antibodies in a dose-dependent manner whereas anti-FVIIa-Gla antibody did not block the binding (Figure 3). Moreover, Fab fragments of anti-FVIIa-EGF2 also inhibited the binding (not shown). This indicates that the EGF1 and EGF2 domains of rFVIIa but not the Gla-domain are important for binding to GPIba as measured by ELISA.

Example 4

Binding between rFVIIa and glycocalicin using surface plasmon resonance analysis

The glycosylated stalk region of GPIba (amino acid 291 - 485) and not the globular head (amino acid 1 - 290) is involved in binding of rFVIIa to the extracellular part of GPIba. This was demonstrated in binding studies were performed using surface plasmon resonance (SPR) analysis as described previously (Lenting PJ et al. J Biol Chem 274: 23734 - 23739, 1999). Glycocalicin, recombinant fragment of GPIba (amino acid 1-290) (Huizinga EG et al. Science 297(5584): 1176-1179, 2002) or rFVIIa was immobilized on a CM5-sensor chip using BIAcore2000 biosensor system and amine-coupling kit as prescribed by the supplier (Biacore AB, Breda, The Netherlands). The immobilisation to the chip was done in sodium acetate pH of 5.0. Prior to the immobilisation the chip was activated with 1-ethyl-3-(3-dimethylaminopropyl) carbodiimide hydrochloride/ N-hydroxysuccinimide (EDC/NHS) for 7 min at 5 ul/min. Immobilisation was done until about 1000 to 1500 RU was coated on the chip. After that the chip was blocked for 7 min at 5 ul/min with ethanolamine HCl. A control chip was routinely activated and blocked in the absence of protein. Binding to coated chips was corrected for binding to non-coated chips. Different concentrations of rFVIIa (on the glycocalicin-coated chip), glycocalicin (on the rFVIIa-coated chip), or a recombinant fragment of GPIba (amino acid 1-290, on the rFVIIa-coated chip) in HEPES buffer (150 mM NaCl, 2 mM $CaCl_2$, 0,005% (v/v) Tween 20, and 20 mM Hepes, pH 7.4) were perfused over the chip with a flow rate of 5 ul/min, and maximal response was evaluated at equilibrium. Regeneration of the sensor chip surface was performed by incubating with 0,1 M Tris and 10 mM taurodeoxycholic acid (Sigma-Aldrich) at pH 9.0. The result showed that rFVIIa bind to immobilized glycocalicin (figure 4A) but not to recombinant GPIba lacking the glycosylated stack (amino acid 1-290, not shown). The Kd value for rFVIIa binding to glycocalicin was determined to approximately 70 nM (figure 4B). The data demonstrate that the glycosylated stack of GPIba is important for rFVIIa binding.

Example 5

Binding of cells expressing GPIba on immobilized rFVIIa

The interaction between GPIba and rFVIIa is confirmed in a cell-based binding assay with CHO cells stabile transfected with human GPIb-IX-V complex (Luo, S-Z et al. Blood, 109(2):603-609, 2007). rFVIIa was immobilized for 2 hrs at 37°C on an Immulon-2 flat-bottom microtiter plate (Dynatech Laboratories Inc, Chantilly, VA) and subsequently blocked with 2% BSA for at least 30 min. CHO-GPIb-IX-V cells (suspended at $10^6$ cells per ml in DMEM:F-12, 0.5% BSA, 1 mM $CaCl_2$, 25 uM $ZnCl_2$) were allowed to adhere for 90 min at 37°C, After extensive washing with Tris-buffered saline, intrinsic phosphatase activity was measured using p-nitrophenol (pNP, 3 mg/ml dissolved in 50 mM acetic acid, 1% Triton X-100, pH 5.0). The reaction was stopped after 30 min with 1 M NaOH and the optical density was measured at 405 nm. The optical density increased linear with the amount of cells adhered. The data showed that wildtype CHO cells did not bind to rFVIIa-covered surface (figure 5A). Only CHO cells transfected with the GPIb-IX-V complex bind to the rFVIIa-covered surface (figure 5A). Moreover, this binding was inhibited by specific mouse monoclonal antibodies against the human rFVII EGF1 (FVII-4F9, IgG1 subclone A106B5), EGF2 (FVII-4F7, IgG1 subclone A4B101) and the Gla-domain (FVII-3F1 IgG1 subclone A2B1C4), (not shown). Furthermore, Fab fragments of anti-FVIIa-EGF2 also inhibited the binding (not shown). Experiments showed that CHO-GPIb-IX-V cells bind to immobilized zymogen FVII but did not bind to rFVIIa-des-Gla (figure 5B). Thus, the cell-based assay confirms the interaction between GPIba and rFVIIa and that this interaction is dependent on the FVIIa-EGF-domains. In contrast to the results obtained by ELISA and Biacore, the cell-based assay also indicates a role of the FVIIa-Gla domain.

Example 6

Binding of rFVIIa to GPIba measured in a static platelet adhesion assay

rFVIIa was immobilized for 2 hrs at 37°C on an Immulon-2 flat-bottom microtiter plate (Dynatech Laboratories Inc, Chantilly, VA) and subsequently blocked with 2% BSA for at least 30 min. Human platelets were isolated from whole blood as described previously (Lisman T et al. J Thromb Haemost 3: 742-751, 2005). Washed platelets (200,000 cells/ μl), in Hepes Tyrode buffer, 10 mM HEPES, 137 mM NaCl, 2.68 mM KCl, 0.42 mM $NaH_2PO_4$, 1.7 mM $MgCl_2$, 5 mM D-glucose, pH 7.4) were activated with either vehicle, thrombin receptor activating peptide (TRAP, 15 uM, Bachem H-2936), or collagen (4 ug/ml collagen Horm, Nycomed, Munich, Germany) and were allowed to adhere for 90 min at 37°C. After extensive washing, intrinsic phosphatase activity was measured using PNP as described in example 5. In some experiments were GPIba removed from the surface of the platelets by preincubating platelets 30 min at 37°C with 5 ug/ml NK, a GPIba-cleaving metalloproteinase purified from cobra (Naja kaouthia, NK) venom (Sigma, St. Louis, MO) using the method previously described for mocarhagin (Ward C et al. Biochemistry 35: 4929-4938, 1996) by Dr R.K. Andrews Monash University, Clayton, Australia. The results showed that only activated platelets bind to immobilized rFVIIa (Figure 6). Moreover, this binding was inhibited by pre-treatment with NK. The data demonstrates that even though the GPIba is constitutively expressed on the surface of the platelets, activation of the platelets is necessary for binding to immobilized rFVIIa. It has previously been shown that activation of platelets is associated with exposure of specific sugar residues such as β-N-acetylglucosamine residues of N-linked glycans on GPIba (Hoffmeister KM et al., Science 301: 1531-1534, 2003), which may facilitate binding of rFVIIa. Moreover, activation of platelets is coupled with exposure of phosphatidylserine on the outer membrane which might be essential for the initial association of rFVIIa via the Gla-domain to the cell membrane followed by a specific binding to GPIba

Example 7

Binding of rFVIIa to platelets as determined by flow cytometry.

Platelets are isolated from platelet-rich plasma by gel filtration and stimulated with thrombin (5 nM) and convulxin (100 ng/ml) in the absence or presence of 100 nM rFVIIa as described (Kjalke M et al. J Thromb Haemost 2007 doi: 10.1111/j.1538-7836.2007.02389.x) or left non-stimulated. Bound rFVIIa variant are detected by flow cytometry using Alexa Flour 488-labelled anti-rFVIIa IgG as described (Kjalke M et al. J Thromb Haemost 2007 doi: 10.1111/j. 1538-7836.2007.02389.x). The GPIba specificity is evaluated by adding an excess GC or by removing GPIba from the surface by proteolytic cleavage using NK as described in example 6. After either of these treatments the rFVIIa variant with enhanced binding to platelets shows similar binding to the platelets as seen for wt rFVIIa.

Example 8

TF-independent Factor X activation on activated platelets.

Washed platelets (Lisman T et al. J Thromb Haemost 3: 742-751, 2005) or platelets isolated from platelet-rich-plasma by gel filtration (Kjalke M et al. J Thromb Haemost 2007 doi: 10.1111/j.1538-7836.2007,02389,x) are activated with TRAP (100 uM, Bachem H-2936), collagen (4 ug/ml collagen Horm Nycomed, Linz, Austria), convulxin (100 ng/ml, Pentapharm), TRAP (100 uM) combined with convulxin (100 ng/ml, Pentapharm), or left non-stimulated. Platelets (final density 100,000/ul) are added to microtiter plates and mixed with rFVIIa or rFVIIa variant (final concentration 31 nM - 2 uM) and factor X (final concentration 0.8 uM) in 100 ul Tyrodes buffer (15 mM hepes, 138 mM NaCl, 2.7 mM KCl, 1 mM MgCl$_2$, 5.5 mM dextrose and 1, mg/ml BSA, pH 7.4.) with 5 mM CaCl$_2$.After 20 min incubation at 37°C with gentle shaking are the factor X activation stopped by adding 50 ul 20 mM EDTA in 20 mM hepes, 150 mM NaCl, pH 7.4. Factor Xa substrate (Chromogenix S-2765, final concentration 0.5 mM) is then added in 50 ul, and colour development followed by measuring absorbance at 405 nm in a Spectramax plate reader. The absorbance at 405 nm can be converted to nM factor Xa generated by the use of a standard curve made by measuring the colour development of samples containing 100 ul factor Xa dilutions (0.07 - 16 nM) mixed with 50 ul 20 mM EDTA in 20 mM hepes, 150 mM NaCl, pH 7.4 and 50 ul factor Xa substrate as described above. Figure 7 shows that rFVIIa dose-dependently increases the activation of factor X on dual activated platelets.

Example 9

The functional consequence of the enhanced binding is further evaluated in a thrombin generation assay using activated platelets

Platelets were isolated from platelet-rich plasma as described previously (Kjalke M et al. Thromb Haemost 78: 1002-1008, 1997) and activated by incubation for 15 min at 37°C with 50 μg/ml of the thrombin receptor agonist peptide SFLLRN (Bachem). Plasma concentrations of prothrombin (1.2 μM), Factor X (135 nM), Factor V (7 μg/ml), ATIII (2.5 μM) and TFPI (0.1 μg/ml) were mixed with CaCl$_2$ (3 mM) and 50 nM of the rFVIIa variants and added to the platelets. Aliquots of 10 μl were removed at timed intervals and added to 90 μl of 1 mM EDTA, 0.5 mM Chromozym TH (Boehringer Mannheim), and 50 μM Pefabloc Xa (Pentapharm) in 20 mM hepes, pH 7.4, 150 mM NaCl, 1 mg/ml BSA. After an appropriate colour development (e.g. after 15 min) the reaction was stopped by addition of 100 μl 50 % (v/v) acetic acid, and the absorbance at 405 nm measured. The colour development by dilutions of a-thrombin was used for a standard curve to convert the absorbance to nM thrombin. Figure 8 shows that rFVIIa dose and time-dependent increase in the thrombin generation on activated platelets.

Example 10

Preparation of rFVIIa glycovariants

Glycans in rFVIIa may be involved in the binding of rFVIIa to GPIba.

Factor VII Preparations Having a Predetermined Pattern of Oligosaccharides. N-glyco varianter:

The methods for producing a preparation of Factor VII comprising any of the glycoform patterns described above as (i)-(vi) and methods for optimizing the glycoform distribution of Factor VII and Factor VII-related polypeptides may be carried out by the steps of:

(a) culturing a cell expressing Factor VII or Factor VII-related polypeptides under a first set of predetermined culture conditions;
(b) recovering Factor VII or FactorVII-related polypeptides from the culture to obtain a preparation comprising the polypeptides; and
(c) analyzing the structure of the oligosaccharides linked to the polypeptides to determine a glycoform pattern.
(d2) treating the preparation chemically and/or enzymatically to alter the oligosaccharide structure; and
(e2) repeating steps (b)-(d2) until a desired glycoform pattern is achieved.

The enzymatic treatments that may be used in step (d2) to modify the oligosaccharide pattern of a preparation include, without limitation, treatment with one or more of sialidase (neuraminidase), galactosidase, fucosidase; galactosyl transferase, fucosyl transferase, and/or sialyltransferase, in a sequence and under conditions that achieve a desired modification in the distribution of oligosaccharide chains having particular terminal structures. Glycosyl transferases are commercially available from Calbiochem (La Jolla, CA) and glycosidases are commercially available from Glyko, Inc., (Novato, CA).

O-glycoforms of rFVIIa with exclusively glucose at serine 52:

The O-glycoforms of rFVIIa with exclusively glucose at serine 52 are obtained by incubation of rFVIIa in an appropriate buffer, e.g. glycylglycine or 20 mM Tris HCl pH 7.0, 2 mM $CaCl_2$, with purified $\alpha$-xylosidase for an appropriate time, that can be determined experimentally. Mass spectra visualizing the deglycosylation are obtained by analysing rFVII $\alpha$-xylosidase incubations ESI-MS (Q-STAR).
The resulting glycan-remodeled rFVIIa is purified from the $\alpha$-xylosidase enzyme by for example anion-exchange chromatography or gel filtration or suitable combinations. The purity of the prepared O-glycoform of rFVIIa is verified by the O-glycopeptide map of rFVIIa.

O-glycoforms of rFVIIa with exclusively xylose-xylose-glucose- at serine 52:

The O-glycoforms of with xylose-xylose-glucose at serine 52 are obtained by (1) treatment of rFVIIa with xylosidase as described above, (2) purification of the xylosidase treated rFVIIa from the xylosidase by for example anion-exchange chromatography, (3) further treatment with UDP-D-xylose: $\beta$-D-glucoside $\alpha$-1,3-D-xylosyltransferase and UDP-D-xylose as described above, and (4) by incubation of the product in an appropriate buffer, e.g. glycylglycine, pH 7.0, 10 mM calcium chloride, with purified UDP-D-xylose: $\alpha$-D-xyloside $\alpha$1,3-xylosyltransferase and UDP-D-xylose for an appropriate time, that can be determined experimentally. The resulting glyco-remodelled rFVIIa is purified from the UDP-D-xylose: $\alpha$-D-xyloside $\alpha$1,3-xylosyltransferase enzyme by for example anion-exchange chromatography. The purity of the prepared O-glycoform of rFVIIa is verified by the O-glycopeptide map of rFVIIa.

Purification of Glc-O-Ser52-FVII and Xyl-Xyl-Glc-O-Ser52-FVII:

Gic-O-ser52-FVII and Xyl-Xyl-Glc-O-Ser52-FVII was purified using two cycles of hydrophobic interaction chromatography (HIC). The column (1.0 cm in inner diameter x 7.0 cm length = 5.5 ml column volume (CV)) packed with Toso Haas TSK-Gel phenyl 5 PW, was equilibrated with 5 CV 10 mM histidine, 10 mM CaCl2, 2.0 M NH4-acetate, pH 6.0. The column was loaded with approximately 2.5 mg of FVII pr. ml resin. To the load solution 2.0 M NH4-acetate and 10 mM CaCl2 was added prior to load. The column was washed with 5 CV 10 mM histidine, 10 mM CaCl2, 2.0 M NH4-acetate, pH 6.0. Elution was performed using a 20 CV linear gradient from 10 mM histidine, 10 mM CaCl2, 2.0 M NH4-acetate, pH 6.0 to 10 mM histidine, 10 mM CaCl2, pH 6.0. The purification was performed at a flow rate of 6 CV/h and at a temperature of 5°C. Fractions were collected during elution.
The FVII eluted in two overlapping major peaks. Fractions containing the first peak were pooled and further purified by a second cycle of HIC, using the same chromatographic procedure as for the first HIC cycle. Fractions containing the second major peak were pooled as well and further purified by a second cycle of HIC, using the same chromatographic procedure as for the first HIC cycle.
Purified Glc-O-Ser52-FVII was identified in the peak fraction, fraction 10, obtained by reloading fraction "A" onto the second HIC step. Purified Xyl-Xyl-Glc-O-Ser52-FVII was identified in the peak fraction, fraction 15, obtained by reloading fraction "B" onto the second HIC step. The identification was obtained by tryptic peptide mapping of rFVIIa and by total mass analysis of rFVIIa. Both assays as described in International patent application WO 2005/111225. Both analyses showed a high content of Glc-O-Ser52-rFVIIa and a low content of Xyl-Xyl-Glc-O-Ser52-rFVIIa in the peak fraction, Fraction 10, and a low content of Glc-O-Ser52-rFVIIa and a high content of Xyl-Xyl-Glc-O-Ser52-rFVIIa in the peak fraction, Fraction 15. A quantitation of the content of the O-glycoforms in the two peak fractions could not be obtained due to relatively low rFVIIa content in the fractions. Tryptic peptide mapping: Other peptide fragments of rFVIIa co-eluted with or eluted close to the O-glycopeptides, and the content of O-glycopeptides in low amounts could therefore not be determined. Total mass analysis: Other O- and/or N-glycoforms of rFVIIa, for example N-glycoforms of rFVIIa lacking one N-acetylneuraminic acid, appeared in the mass spectra, and the content of O-glycoforms of rFVIIa in low amounts could therefore not be determined.

The specific activities of the peak fractions obtained from the HIC were determined by the 1st generation clotting assay.

The enhanced binding affinity of the FVII polypeptides described above are studied in the screening assays described in example 14. The data is validated using cell adhesion as described in example 7 and 8, and the functional consequence determined in the assays measuring TF-independent factor X activation and thrombin generation described in example 10 and 11, respectively.

Example 11

rFVIIa mutants

Polypeptide variants of rFVIIa are generated by random mutagenesis in selected regions of rFVIIa. Results suggest that the EGF1 and EGF2 domains of rFVIIa are important for binding to GPIba as measured by ELISA and in the cell-based binding assay (described in example 3 and 5, respectively). Moreover, data suggest that rFVIIa bound to soluble tissue factor also associates with GPIba measured in the cell-based binding assay with CHO cells stabil transfected with human GPIb-IX-V complex as described in example 5. This part of the invention relates to polypeptide variants of rFVIIa, which comprise an amino acid substitution or amino acid substitutions in the EGF1 and EGF2 domain of the light chain of rFVIIa. The amino acid positions being modified will preferably be taken from the pool of side chains of the EGF1 and EGF2 domains having a relative surface accessibility > 40% as estimated from the x-ray crystallographic structure of human rFVIIa in complex with soluble TF (The Protein Data Bank entry: 1dan. Reference: H.M. Berman et al. The Protein Data Bank), Nucleic Acids Research, 28 pp. 235-242 (2000). Hence, side chains in close contact to soluble TF are not considered. The list is given below where ASA1 is the total accessible of the residue alone in the conformation in the given structure in Angstroms squared. ASA2 is the total accessible surface area (as seen by e.g. a water molecule) in Angstroms squared. Rel. ASA is ASA2 divided by the maximal accessible surface area of the side chain in question (which is not necessarily ASA1).

Light chain, Gla domain excluded:

| Res.no | Residue | ASA1 | ASA2 | Rel ASA |
|--------|---------|--------|--------|---------|
| L:38 | LYS | 134.00 | 131.50 | 0.69 |
| L:42 | ILE | 77.89 | 70.27 | 0.40 |
| L:47 | GLY | 43.61 | 39.68 | 0.80 |
| L:48 | ASP | 80.49 | 68.99 | 0.67 |
| L:51 | ALA | 81.84 | 50.29 | 0.55 |
| L:53 | SER | 92.47 | 76,59 | 0,85 |
| L:56 | GLN | 92.21 | 82.66 | 0.55 |
| L:58 | GLY | 46.25 | 38.36 | 0.77 |
| L:66 | GLN | 145.78 | 124.40 | 0.83 |
| L:67 | SER | 53.90 | 49.14 | 0.55 |
| L:73 | LEU | 76.94 | 76.14 | 0.42 |
| L:74 | PRO | 76.28 | 69.95 | 0.66 |
| L:75 | ALA | 44.03 | 39.46 | 0.43 |
| L:84 | HIS | 79.08 | 77.03 | 0.51 |
| L:86 | ASP | 118.24 | 95.70 | 0.93 |
| L:87 | ASP | 106.76 | 80.81 | 0,78 |
| L:89 | LEU | 81.84 | 72.73 | 0.40 |
| L:94 | GLU | 90.85 | 81.25 | 0.54 |

(continued)

| Res.no | Residue | ASA1 | ASA2 | Rel ASA |
|---|---|---|---|---|
| L:97 | GLY | 36.61 | 25.91 | 0.52 |
| L:104 | ASP | 67.84 | 49.00 | 0.47 |
| L:105 | HIS | 88.23 | 67.57 | 0.44 |
| L:106 | THR | 150.16 | 127.98 | 1.01 |
| L:107 | GLY | 86.22 | 47.25 | 0.95 |
| L:108 | THR | 101.23 | 76.64 | 0.61 |
| L:109 | LYS | 148.89 | 130.91 | 0.69 |
| L:111 | SER | 44.13 | 39.78 | 0.44 |
| L:113 | ARG | 92.40 | 88.49 | 0.42 |
| L:116 | GLU | 128.06 | 112.38 | 0.75 |
| L:119 | SER | 54.90 | 49.31 | 0.55 |
| L:121 | LEU | 84.11 | 76.41 | 0.42 |
| L:122 | ALA | 113,00 | 69.68 | 0.76 |
| L:123 | ASP | 82.35 | 53.24 | 0.51 |
| L:124 | GLY | 30.58 | 28.73 | 0.58 |
| L:128 | THR | 51.29 | 51.00 | 0.40 |
| L:129 | PRO | 60.05 | 42.02 | 0.40 |
| L;130 | THR | 89.11 | 50.55 | 0.40 |
| L:131 | VAL | 79.05 | 63.34 | 0.46 |
| L:132 | GLU | 144.31 | 117.18 | 0.79 |
| L:142 | GLU | 161.66 | 157.27 | 1.05 |
| L:143 - L:152 | | | | |

The screening candidates described above will be studied in the screening assays described in example 14, The functional consequence determined in the assays measuring TF-independent factor X activation and thrombin generation described in example 8 and 9, respectively.

Example 12

In Vitro Hydrolysis Assay - test of rFVIIa variants.

Native (wild-type) Factor VIIa and Factor VIIa variant (both hereafter referred to as "Factor VIIa") are assayed in parallel to directly compare their specific activities. The assay is carried out in a microtiter plate (MaxiSorp, Nunc, Denmark). The chromogenic substrate D-Ile-Pro-Arg-p-nitroanilide (S-2288, Chromogenix, Sweden), final concentration 1 mM, is added to Factor VIIa (final concentration 10 nM) and the extracellular domain of TF (sTF (Freskgaard PO et al. Protein Sci 5: 1531-40, 1996), final concentration 10 nM) in 50 mM Hepes, pH 7.4, containing 0.1 M NaCl, 5 mM $CaCl_2$ and 1 mg/ml bovine serum albumin. The absorbance at 405 nm is measured continuously in a SpectraMax™ 340 plate reader (Molecular Devices, USA). The absorbance developed during a 20-minute incubation, after subtraction of the absorbance in a blank well containing no enzyme, is used to calculate the ratio between the activities of variant and wild-type Factor VIIa:
Ratio = (A405 nm Factor VIIa variant)/(A405 nm Factor VIIa wild-type).

Example 13

In Vitro Proteolysis Assay - test of rFVIIa variants.

Native (wild-type) Factor VIIa and Factor VIIa variant (both hereafter referred to as "Factor VIIa") are assayed in parallel to directly compare their specific activities. The assay is carried out in a microtiter plate (MaxiSorp, Nunc, Denmark). Factor VIIa (0.2 nM), and relipidated TF (Americaln Diagnostica product no 4500L or similar, 0.02 nM) in 50 ul 50 mM Hepes, pH 7.4, containing 0.1 M NaCl, 5 mM $CaCl_2$ and 1 mg/ml bovine serum albumin, are incubated for 15 min before addition of 50 μl 100 nM Factor X Factor X cleavage is then stopped after 10 min by the addition of 50 uL 50 mM Hepes, pH 7.4, containing 0.1 M Nacl, 20 mM EDTA and 1 mg/ml bovine serum albumin. The amount of Factor Xa generated is measured by addition of the chromogenic substrate Z-D-Arg-Gly-Arg-p-nitroanilide (S-2765, Chromogenix, Sweden), final concentration 0.5 mM. The absorbance at 405 nm is measured continuously in a SpectraMax™ 340 plate reader (Molecular Devices, USA). The absorbance developed during 10 minutes, after subtraction of the absorbance in a blank well containing no rFVIIa, is used to calculate the ratio between the proteolytic activities of variant and wild-type Factor VIIa:

Ratio = (A405 nm Factor VIIa variant)/(A405 nm Factor VIIa wild-type).

Example 14

Screening plan

rFVIIa variants with an amidolytic and proteolytic activity at least similar to rFVIIa determine in the in vitro hydrolysis assay and proteolysis assay described in example 12 and 13, respectively, will be included in the screening plan. Screening methods for identification of rFVIIa variants with enhanced TF-independent activity due to enhanced binding to GPIba involves the following five assays:

Screening assay 1 using surface plasmon resonance with immobilised glycocalicin:

Biacore assay with glycocalicin immobilised on a CM5-sensor chip is carried out as described in example 6. Briefly, glycocalicin purified from plasma as described in example 4 are immobilized on a CM5-sensor chip at a density of 1000 - 1500 RU using amine-coupling. After blockage different concentrations of rFVIIa or rFVIIa variants are perfused over the chip at a flow of 5 ul/min as described in example 6, and maximal response are evaluated at equilibrium. The rFVIIa variant should have improved binding affinity with at least 20 % lower Kd for binding to glycocalicin than that of wildtype rFVIIa

Screening assay 2 using ELISA with glycocalicin-coated microtiter plates:

Binding to glycocalicin-coated microtiter plates is performed as decribed in example 4. Briefly, glycocalicin (2 ug/ml) purified from plasma as in example 4 is allowed to adhere to immobilized rFVIIa or rFVIIa variants as described in example 4. After blocking the wells bound glycocalicin are detected with a rabbit polyclonal antibody against GPIb and a peroxidase-labeled swine-anti-rabbit antibody, followed by TNB staining. Results are obtained by measuring optical density at 490 nm on a SpectraMax Reader. Increased binding is defined as at least 20 % enhanced binding affinity compared to wild-type rFVIIa.

Screening assay 3 using GPIb-V-IX-transfected CHO cells:

Binding of GPIb-V-IX-transfected CHO cells to rFVIIa variants is analyzed using immobilized rFVIIa variants as described in example 5. After blocking are CHO-GPIb-IX-V cells allowed to adhere and bound cells detected after extensive washing using PNP as described in example 5. Increased binding in the screening assays is defined as at least 20 % enhanced binding affinity compared to wild-type rFVIIa.

Screening assay 4 using static platelet adhesion:

Binding of activated platelets to rFVIIa variants is analyzed using immobilized rFVIIa variants as described in example 6. After blocking are activated platelets allowed to adhere and bound cells detected after extensive washing using PNP as described in example 6. Increased binding in the screening assays is defined as at least 20 % enhanced binding affinity compared to wild-type rFVIIa.

Screening assay 5 using flow cytometry

Binding of rFVIIa or rFVIIa variants to activated platelets is determined by flow cytometry as described in example 7. Briefly, platelets are activated in the presence of 100 nM rFVIIa or rFVIIa variants and bound rFVIIa or rFVIIa variant detected by flow cytometry using Alexa Flour 488-labelled anti-rFVIIa IgG as described (Kjalke M et al. J Thromb Haemost 2007 doi: 10.1111/j.1538-7836.2007,02389.x).

rFVIIa variants with increased binding to GPIba in one of the screening assays are validated using the functional assays measuring TF-independent factor X activation and thrombin generation described in example 8 and 9, respectively.

Example 15

A method to identify rFVIIa "high-responder" patients.

Individuals with high level of GPIba on (activated) platelets is expected to bind more rFVIIa and consequently be "high-responders" of rFVIIa therapy, i.e. will require lower doses of rFVIIa for haemostatic efficacy. This can be evaluated by screening patient platelets for GPIba expression e.g. by flow cytometry experiments using either a whole blood sample or isolated platelets. Samples containing $10^6$ platelets are incubated with excess fluorophor-labelled anti-GPIba antibody e.g. FITC or PE-labelled CD42b from Pharmingen (cat no. 555472 or 555473) or from Immunotech (BeckmanCoulter IM0648 or IM1417) 10 min in the dark, and the cells fixed by adding 1% paraformaldehyde. In case whole blood is used erythrocytes are lyzed using e.g. FACSlysis (BD) as described by the manufactures. The fluorescence intensity is determined by flow cytometry using e.g. a FACSCanto instrument (BD). Forward and side scatter light channels and fluorescence channels are set on log. Platelets are identified by their forward and side scatter characteristics or by using an alternative anti-platelet antibody e.g. CD61 labeled with a fluorophor as marker. The number of GPIba molecules per platelet is determined by the use of calibration beads (Rainbow calibration particles, 8 peaks, Sphero, BD) with a known amount of fluorescence per bead as described by the manufacturer. Initially, platelets from patients known to respond well or poorly to rFVIIa therapy (Sørensen B and Ingerslev J, J Thromb Haemost 2: 102-110, 2004) can be analyzed for GPIba content and rFVIIa binding as described in (Kjalke M et al. J Thromb Haemost 2007 doi: 10.1111/j.1538-7836,2007.02389.x). If the known phenotype of the patients (high/low responders) correlate with GPIba content and rFVIIa binding quantification of GPIba density and/or rFVIIa binding to the platelets can be used to predict the rFVIIa doses required for obtaining a haemostatic effect in the patient.

Example 16

Prolonged half-life of rFVIIa.

A rFVIIa variant with enhanced affinity to GPIba may bind platelets independently of phosphatidylserine exposure and therefore exists in vivo longer than wildtype rFVIIa due to circulation of the rFVIIa variant bound to platelets. Upon vascular injury the platelets will be activated and the GPIba-associated rFVIIa variant will be available for enhancing thrombin generation locally on the activated platelet thereby providing a haemostatic effect. As long as the platelets are not activated no risk of systemic coagulation is expected. Rather the GPIba-bound rFVIIa variant constitutes a pool of rFVIIa readily available for a haemostatic effect. The functional life span for the rFVIIa variant may therefore be extended up to the entire life-span of the platelet, i.e. 10 days. This is evaluated in a time and efficacy study using FVIII-deficient mice, where wildtype rFVIIa and rFVIIa variant will be administrated at different time-points prior to tail clipping and evaluation of total bleeding time and blood loss as described (Tranholm M et al. Blood 102: 3615-3620, 2003). Briefly, wildtype rFVIIa and rFVIIa variant in a dose of e.g. 15 mg/kg are administrated i.v. to FVIII-deficient mice and tail clipping performed after 5, 16, 40, 60, 80, and 180 min. Later timepoints e.g. 20 hrs may be included. Wildtype rFVIIa showed a significant reduction of total bleeding time after 5 and 16 min as illustrated in figure 9. The rFVIIa variant with enhanced affinity to GPIba will shorten the tail bleeding time and decreased the blood loss for extended timepoints compared to wild-type rFVIIa.
The presence of rFVIIa variant on the platelets for extended time points are evaluated by flow cytometry where wildtype rFVIIa and rFVIIa variant (4 or 15 mg/kg) are administrated to FVIII-deficient or normal mice and the rFVIIa or rFVIIa variant bound to platelets measured at various time-points by flow cytometry. Briefly, eye blood is isolated by the use of a 1 ul capillary glass tube and added to 1/10 vol 10 uM Tick Anticoagulant Peptide (Corvas) and 68 uM hirudin (Enzyme Research) before rFVIIa administration and 30 min, 1 hr, 2 hr, 4 hr, 6 hr and 20 hrs after administration (some time points may be omitted), The blood is mixed with 1 ml FACS lysing solution (BD) with 5 mM $CaCl_2$ added, and the erythrocytes lysed for 10 min at room temperature, and the platelets isolated by 5 min centrifugation at 200 x g. The platelets are resuspended in Tyrodes buffer with 5 mM $CaCl_2$ and counted e.g. by flow cytometry as described (Kjalke M et al. J Thromb Haemost 2007 doi: 10.1111/j.1538-7836.2007.02389.x). Platelets ($10^6$ platelets/sample) are labeled with 1 ug Alexa Fluor 647-labeled anti-mouse CD61 and Alexa Flour 488-labeled anti-rFVIIa IgG as described (Kjalke M et al. J Thromb Haemost 2007 doi: 10.1111/j.1538-7836.2007.02389.x). The Alexa Fluor 647-labeling of anti-mouse CD61 (BD Pharmingen no 553344) are performed using Alexa Fluor 647 protein labeling kit (Molecular probes # A20173) according to the manufacturers instructions. After 60 min incubation in the dark are cells washed once with 1 ml HBS buffer with 1 mg/ml BSA and 5 mM $CaCl_2$ and resuspended in 1 ml HBS buffer with 5 mM $CaCl_2$. The samples are analyzed for rFVIIa binding as described for human platelets (see example 7). Wildtype rFVIIa could be detected on the platelets after 15 min and to a lower extend after 4 hrs as illustrated in figure 10. The rFVIIa variants with enhanced affinity to GPIba can be detected in increased amounts seen as increased fluorescence intensity of anti-rFVIIa IgG and/or for an extended time compared to wildtype rFVIIa.

**Claims**

1. A method for the identification of a FVII polypeptide with increased binding affinity to activated human platelets as compared to human wild-type FVIIa, said method comprising the steps of:

   a) providing a FVII polypeptide different from human wild-type FVIIa;
   b) testing said FVII polypeptide different from human wild-type FVIIa for the binding affinity to human platelet glycoprotein Ib alpha;
   c) testing said FVII polypeptide different from human wild-type FVIIa for functional coagulation activity;
   d) selecting said FVII polypeptide with both i) functional coagulation activity substantially the same as human wild-type FVIIa; and ii) increased binding affinity of said FVII polypeptide to human platelet glycoprotein Ib alpha as compared to human wild-type FVIIa.

2. The method according to claim 1, wherein said binding affinity of said FVII polypeptide is measured by any method selected from the list consisting of:

   a) binding of said FVII polypeptide to CHO cells transfected with human glycoprotein Ib alpha;
   b) binding of said FVII polypeptide to activated platelets, such as in a static platelet adhesion assay, or a flow cytometry assay;
   c) binding of said FVII polypeptide to immobilised Glycocalicin, such as in a Biacore or ELISA assay; and
   d) binding of Glycocalicin to an immobilized preparation of said FVII polypeptide, such as in a Biacore or ELISA assay.

3. The method according to any of claims 1 or 2, wherein said functional coagulation activity of said FVII polypeptide is measured by any assay selected from

   a) in vitro hydrolysis assay;
   b) in vitro proteolysis assay;
   c) FX activation on activated platelets and
   d) thrombin generation assay on activated platelets

4. The method according to any one of claims 1-3, wherein said affinity of said FVII polypeptide is 10 % higher than that of human wild-type FVIIa, such as 20 % higher than that of human wild-type FVIIa, such as 30 % higher than that of human wild-type FVIIa, such as 40 % higher than that of human wild-type FVIIa. such as 50 % higher than that of human wild-type FVIIa. such as 100 % higher than that of human wild-type FVIIa.

5. The method according to any one of claims 1-4, wherein said FVII polypeptide is provided by random mutagenesis, site-directed mutagenesis, or modification of glycostructure.

6. The method according to any one of claims 1-5, wherein said FVII polypeptide comprises one or more substitutions relative to the amino acid sequence of SEQ ID NO: 1 in any one of the EGF-like domain 1 or EGF-like domain 2 of said FVIIa polypeptide.

7. The method according to claim 6, wherein said substitution is in one or more position selected from the list consisting of Lys38, Ile42, Gly47, Asp48, Ala51, Ser53, Gln56, Gly58, Gln66, Ser67, Leu73, Pro74, Ala75, His84, Asp86, Asp87, Leu89, Glu94, Gly97, Asp104, His105, Thr106, Gly107, Thr108, Lys109, Ser111, Arg113, Glu116, Ser119, Leu121, Ala122, Asp123, Gly124, Thr128, Pro129, Thr130, Val131, Glu132, and Glu142.

8. A FVII polypeptide obtainable by a method according to any of claims 1-7.

9. The FVII polypeptide according to claim 8, which Factor VII polypeptide has a modified structure of N-linked and/or O-linked oligosaccharides.

10. The FVII polypeptide according to any one of claims 8-9, further comprising amino acid substitutions selected from the list consisting of:

L305V, L305V/M306D/D309S, L305I, L305T, F374P, V158T/M298Q, V158D/E296V/M298Q, K337A, M298Q, V158D/M298Q, L305V/K337A, V158D/E296V/M298Q/L305V, V158D/E296V/M298Q/K337A, V158D/E296V/M298Q/L305V/K337A, K157A, E296V, E296V/M298Q, V158D/E296V, V158D/M298K, and S336G, L305V/K337A, L305V/V158D, L305V/E296V, L305V/M298Q, L305V/V158T, L305V/K337A/V158T, L305V/K337A/M298Q, L305V/K337A/E296V, L305V/K337A/V158D, L305V/V158D/M298Q, L305V/V158D/E296V, L305V/V158T/M298Q, L305V/V158T/E296V, L305V/E296V/M298Q, L305V/V158D/E296V/M298Q, L305V/V158T/E296V/M298Q, L305V/V158T/K337A/M298Q, L305V/V158T/E296V/K337A, L305V/V158D/K337A/M298Q, L305V/V158D/E296V/K337A, L305V/V158D/E296V/M298Q/K337A, L305V/V158T/E296V/M298Q/K337A, S314E/K316H, S314E/K316Q, S314E/L305V, S314E/K337A, S314E/V158D, S314E/E296V, S314E/M298Q, S314E/V158T, K316H/L305V, K316H/K337A, K316H/V158D, K316H/E296V, K316H/M298Q, K316H/V158T, K316Q/L305V, K316Q/K337A, K316Q/V158D, K316Q/E296V, K316Q/M298Q, K316Q/V158T, S314E/L305V/K337A, S314E/L305V/V158D, S314E/L305V/E296V, S314E/L305V/M298Q, S314E/L305V/V158T, S314E/L305V/K337A/V158T, S314E/L305V/K337A/M298Q, S314E/L305V/K337A/E296V, S314E/L305V/K337A/V158D, S314E/L305V/V158D/M298Q, S314E/L305V/V158D/E296V, S314E/L305V/V158T/M298Q, S314E/L305V/V158T/E296V, S314E/L305V/E296V/M298Q, S314E/L305V/V158D/E296V/M298Q, S314E/L305V/V158T/E296V/M298Q, S314E/L305V/V158T/K337A/M298Q, S314E/L305V/V158T/E296V/K337A, S314E/L305V/V158D/K337A/M298Q, S314E/L305V/V158D/E296V/K337A, S314E/L305V/V158D/E296V/M298Q/K337A, S314E/L305V/V158T/E296V/M298Q/K337A, K316H/L305V/K337A, K316H/L305V/V158D, K316H/L305V/E296V, K316H/L305V/M298Q, K316H/L305V/V158T, K316H/L305V/K337A/V158T, K316H/L305V/K337A/M298Q, K316H/L305V/K337A/E296V, K316H/L305V/K337A/V158D, K316H/L305V/VI58D/M298Q, K316H/L305V/V158D/E296V, K316H/L305V/V158T/M298Q, K316H/L305V/V158T/E296V, K316H/L305V/E296V/M298Q, K316H/L305V/V158D/E296V/M298Q, K316H/L305V/V158T/E296V/M298Q, K316H/L305V/V158T/K337A/M298Q, K316H/L305V/V158T/E296V/K337A, K316H/L305V/V158D/K337A/M298Q, K316H/L305V/VI58D/E296V/K337A, K316H/L305V/V158D/E296V/M298Q/K337A, K316H/L305V/VI58T/E296V/M298Q/K337A, K316Q/L305V/K337A, K316Q/L305V/V158D, K316Q/L305V/E296V, K316Q/L305V/M298Q, K316Q/L305V/V158T, K316Q/L305V/K337A/V158T, K316Q/L305V/K337A/M298Q, K316Q/L305V/K337A/E296V, K316Q/L305V/K337A/V158D, K316Q/L305V/V158D/M298Q, K316Q/L305V/V158D/E296V, K316Q/L305V/V158T/M298Q, K316Q/L305V/V158T/E296V, K316Q/L305V/E296V/M298Q, K316Q/L305V/V158D/E296V/M298Q, K316Q/L305V/V158T/E296V/M298Q, K316Q/L305V/V158T/K337A/M298Q, K316Q/L305V/V158T/E296V/K337A, K316Q/L305V/V158D/K337A/M298Q, K316Q/L305V/VI58D/E296V/K337A, K316Q/L305V/V158D/E296V/M298Q/K337A, K316Q/L305V/V158T/E296V/M298Q/K337A, F374Y/K337A, F374Y/V158D, F374Y/E296V, F374Y/M298Q, F374Y/V158T, F374Y/S314E, F374Y/L305V, F374Y/L305V/K337A, F374Y/L305V/V158D, F374Y/L305V/E296V, F374Y/L305V/M298Q, F374Y/L305V/V158T, F374Y/L305V/S314E, F374Y/K337A/S314E, F374Y/K337A/V158T, F374Y/K337A/M298Q, F374Y/K337A/E296V, F374Y/K337A/V158D, F374Y/V158D/S314E, F374Y/V158D/M298Q, F374Y/V158D/E296V, F374Y/V158T/S314E, F374Y/V158T/M298Q, F374Y/V158T/E296V, F374Y/E296V/S314E, F374Y/S314E/M298Q, F374Y/E296V/M298Q, F374Y/L305V/K337A/VI58D, F374Y/L305V/K337A/E296V, F374Y/L305V/K337A/M298Q, F374Y/L305V/K337A/V158T, F374Y/L305V/K337A/S314E, F374Y/L305V/V158D/E296V, F374Y/L305V/V158D/M298Q, F374Y/L305V/V158D/S314E, F374Y/L305V/E296V/M298Q, F374Y/L305V/E296V/V158T, F374Y/L305V/E296V/S314E, F374Y/L305V/M298Q/VI58T, F374Y/L305V/M298Q/S314E, F374Y/L305V/V158T/S314E, F374Y/K337A/S314E/V158T, F374Y/K337A/S314E/M298Q, F374Y/K337A/S314E/E296V, F374Y/K337A/S314E/V158D,

F374Y/K337A/V158T/M298Q, F374Y/K337A/V158T/E296V, F374Y/K337A/M298Q/E296V, F374Y/K337A/M298Q/V158D, F374Y/K337A/E296V/V158D, F374Y/V158D/S314E/M298Q, F374Y/V158D/S314E/E296V, F374Y/V158D/M298Q/E296V, F374Y/V158T/S314E/E296V, F374Y/V158T/S314E/M298Q, F374Y/V158T/M298Q/E296V, F374Y/E296V/S314E/M298Q, F374Y/L305V/M298Q/K337A/S314E, F374Y/L305V/E296V/K337A/S314E, F374Y/E296V/M298Q/K337A/S314E, F374Y/L305V/E296V/M298Q/K337A, F374Y/L305V/E296V/M298Q/S314E, F374Y/V158D/E296V/M298Q/K337A, F374Y/V158D/E296V/M298Q/S314E, F374Y/L305V/V158D/K337A/S314E, F374Y/V158D/M298Q/K337A/S314E, F374Y/V158D/E296V/K337A/S314E, F374Y/L305V/V158D/E296V/M298Q, F374Y/L305V/V158D/M298Q/K337A, F374Y/L305V/V158D/E296V/K337A, F374Y/L305V/V158D/M298Q/S314E, F374Y/L305V/V158D/E296V/S314E, F374Y/V158T/E296V/M298Q/K337A, F374Y/V158T/E296V/M298Q/S314E, F374Y/L305V/V158T/K337A/S314E, F374Y/V158T/M298Q/K337A/S314E, F374Y/V158T/E296V/K337A/S314E, F374Y/L305V/V158T/E296V/M298Q, F374Y/L305V/V158T/M298Q/K337A, F374Y/L305V/V158T/E296V/K337A, F374Y/L305V/VI58T/M298Q/S314E, F374Y/L305V/V158T/E296V/S314E, F374Y/E296V/M298Q/K337A/V158T/S314E, F374Y/V158D/E296V/M298Q/K337A/S314E, F374Y/L305V/V158D/E296V/M298Q/S314E, F374Y/L305V/E296V/M298Q/V158T/S314E, F374Y/L305V/E296V/M298Q/K337A/V158T, F374Y/L305V/E296V/K337A/V158T/S314E, F374Y/L305V/M298Q/K337A/V158T/S314E, F374Y/L305V/V158D/E296V/M298Q/K337A, F374Y/L305V/V158D/E296V/K337A/S314E, F374Y/L305V/V158D/M298Q/K337A/S314E, F374Y/L305V/E296V/M298Q/K337A/V158T/S314E, F374Y/L305V/V158D/E296V/M298Q/K337A/S314E, S52A, S60A; R152E, S344A, T106N, K143N/N145T, V253N, R290N/A292T, G291N, R315N/V317T, and K143N/N145T/R315N/V317T.

Figure 1

FIGURE 1 - the amino acid sequence of wild-type human coagulation Factor VII (SEQ ID NO:1)


Ala-Asn-Ala-Phe-Leu-GLA-GLA-Leu-Arg-Pro-Gly-Ser-Leu-GLA-Arg-GLA-Cys-Lys-

GLA-GLA-Gln-Cys-Ser-Phe-GLA-GLA-Ala-Arg-GLA-Ile-Phe-Lys-Asp-Ala-GLA-Arg-

Thr-Lys-Leu-Phe-Trp-Ile-Ser-Tyr-Ser-Asp-Gly-Asp-Gln-Cys-Ala-Ser-Ser-Pro-

Cys-Gln-Asn-Gly-Gly-Ser-Cys-Lys-Asp-Gln-Leu-Gln-Ser-Tyr-Ile-Cys-Phe-Cys-

Leu-Pro-Ala-Phe-Glu-Gly-Arg-Asn-Cys-Glu-Thr-His-Lys-Asp-Asp-Gln-Leu-Ile-

Cys-Val-Asn-Glu-Asn-Gly-Gly-Cys-Glu-Gln-Tyr-Cys-Ser-Asp-His-Thr-Gly-Thr-

Lys-Arg-Ser-Cys-Arg-Cys-His-Glu-Gly-Tyr-Ser-Leu-Leu-Ala-Asp-Gly-Val-Ser-

Cys-Thr-Pro-Thr-Val-Glu-Tyr-Pro-Cys-Gly-Lys-Ile-Pro-Ile-Leu-Glu-Lys-Arg-

Asn-Ala-Ser-Lys-Pro-Gln-Gly-Arg-Ile-Val-Gly-Gly-Lys-Val-Cys-Pro-Lys-Gly-

Glu-Cys-Pro-Trp-Gln-Val-Leu-Leu-Leu-Val-Asn-Gly-Ala-Gln-Leu-Cys-Gly-Gly-

Thr-Leu-Ile-Asn-Thr-Ile-Trp-Val-Val-Ser-Ala-Ala-His-Cys-Phe-Asp-Lys-Ile-

Lys-Asn-Trp-Arg-Asn-Leu-Ile-Ala-Val-Leu-Gly-Glu-His-Asp-Leu-Ser-Glu-His-
200                     205                     210                     215

Asp-Gly-Asp-Glu-Gln-Ser-Arg-Arg-Val-Ala-Gln-Val-Ile-Ile-Pro-Ser-Thr-Tyr-
                    220                     225                     230

Val-Pro-Gly-Thr-Thr-Asn-His-Asp-Ile-Ala-Leu-Leu-Arg-Leu-His-Gln-Pro-Val-
235                     240                     245                     250

Val-Leu-Thr-Asp-His-Val-Val-Pro-Leu-Cys-Leu-Pro-Glu-Arg-Thr-Phe-Ser-Glu-
            255                     260                     265                     270

Arg-Thr-Leu-Ala-Phe-Val-Arg-Phe-Ser-Leu-Val-Ser-Gly-Trp-Gly-Gln-Leu-Leu-
            275                     280                     285

Asp-Arg-Gly-Ala-Thr-Ala-Leu-Glu-Leu-Met-Val-Leu-Asn-Val-Pro-Arg-Leu-Met-
        290                     295                     300                     305 306

Thr-Gln-Asp-Cys-Leu-Gln-Gln-Ser-Arg-Lys-Val-Gly-Asp-Ser-Pro-Asn-Ile-Thr-
            310                     315                     320

Glu-Tyr-Met-Phe-Cys-Ala-Gly-Tyr-Ser-Asp-Gly-Ser-Lys-Asp-Ser-Cys-Lys-Gly-
325                     330                     335                     340

Asp-Ser-Gly-Gly-Pro-His-Ala-Thr-His-Tyr-Arg-Gly-Thr-Trp-Tyr-Leu-Thr-Gly-
        345                     350                     355                     360

Ile-Val-Ser-Trp-Gly-Gln-Gly-Cys-Ala-Thr-Val-Gly-His-Phe-Gly-Val-Tyr-Thr-
                365                     370                     375

Arg-Val-Ser-Gln-Tyr-Ile-Glu-Trp-Leu-Gln-Lys-Leu-Met-Arg-Ser-Glu-Pro-Arg-
        380                     385                     390                     395

Pro-Gly-Val-Leu-Leu-Arg-Ala-Pro-Phe-Pro
            400                     405 406

Figure 2

**A**

**B**

Figure 3

Figure 4

**A**

Perfusion of rFVIIa over immobilized glycocalicin

**B**

Perfusion of rFVIIa over immobilized glycocalicin

Figure 5

**A**

CHO static adhesion

**B**

CHO static cell adhesion

Figure 6

Static platelet adhesion

Figure 7

FXa generation on platelets

Figure 8

Thrombin generation on activated platelets

Figure 9

Bleeding time in FVIII-KO mice at various
time-points after rFVIIa administration

Figure 10

## rFVIIa binding to mouse platelets in vivo

Filled histogram: pre-dose (only anti-rFVIIa IgG)
Bold line: 45 min after rFVIIa administration (15 mg/kg)
Thin line: 3 hrs after rFVIIa administration (15 mg/kg)

**European Patent Office**

**EUROPEAN SEARCH REPORT**

Application Number

EP 07 10 1259

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALMEIDA ANTONIO M ET AL: "The use of recombinant factor VIIa in children with inherited platelet function disorders" BRITISH JOURNAL OF HAEMATOLOGY, OXFORD, GB, vol. 121, no. 3, May 2003 (2003-05), pages 477-481, XP009090582 ISSN: 0007-1048 ----- | | INV. A61K38/36 C07K14/745 C12N9/64 G01N33/68 |
| A | MARGARETH CASTRO OZELO ET AL: "Use of recombinant factor VIIa in the management of severe bleeding episodes in patients with Bernard-Soulier syndrome" ANNALS OF HEMATOLOGY, SPRINGER-VERLAG, BE, vol. 84, no. 12, 1 December 2005 (2005-12-01), pages 816-822, XP019333840 ISSN: 1432-0584 ----- | | |
| A | WO 03/027147 A (NOVO NORDISK AS [DK] NOVO NORDISK HEALTH CARE AG [CH]) 3 April 2003 (2003-04-03) ----- | | |
| A | WO 2005/075635 A (NOVO NORDISK HEALTH CARE AG [CH]; OESTERGAARD HENRIK [DK]; PERSSON EGO) 18 August 2005 (2005-08-18) ----- | | TECHNICAL FIELDS SEARCHED (IPC) A61K C07K C12N G01N |
| A | WO 2004/110469 A (NOVO NORDISK AS [DK]; PERSSON EGON [SE]; HANSEN CARSTEN BORGUND [DK]) 23 December 2004 (2004-12-23) ----- | | |
| A | WO 2004/111242 A (MAXYGEN HOLDINGS LTD; MAXYGEN APS [DK]; HAANING JESPER MORTENSEN [DK];) 23 December 2004 (2004-12-23) ----- | | |

~~The present search report has been drawn up for all claims~~

| Place of search | Date of completion of the search | Examiner |
|---|---|---|
| Munich | 16 October 2007 | Vogt, Titus |

EPO FORM 1503 03.82 (P04C01)

European Patent

Office

Application Number

EP 07 10 1259

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

Claims 1-5 and partly 6 and 7

☐ The present supplementary European search report has been drawn up for those parts of the European patent application which relate to the invention first mentioned in the claims (Rule 164 (1) EPC).

**European Patent Office**

**LACK OF UNITY OF INVENTION
SHEET B**

Application Number

EP 07 10 1259

The Search Division considers that the present European patent application does not comply with the requirements of unity of invention and relates to several inventions or groups of inventions, namely:

Invention 1: Claims 1-5 and partly claims 6 and 7.

    Method for the identification of Factor VII derivatives with increased binding affinity towards human platelet glycoprotein Ib alpha.

---

Inventions 2-40: Claims 8-10, and partly claims 6 and 7.

    Factor VII derivatives identified by the method of invention 1.
It is noted that this invention must be further subdivided into a plurality of inventions (at least 39), each directed to a single FVII polypeptide with a modification listed in claim 7.

---

## ANNEX TO THE EUROPEAN SEARCH REPORT
## ON EUROPEAN PATENT APPLICATION NO.

EP 07 10 1259

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2007

| Patent document cited in search report | | Publication date | Patent family member(s) | | Publication date |
|---|---|---|---|---|---|
| WO 03027147 | A | 03-04-2003 | BR | 0212818 A | 05-10-2004 |
| | | | CA | 2461003 A1 | 03-04-2003 |
| | | | CN | 1602354 A | 30-03-2005 |
| | | | CZ | 20040427 A3 | 18-08-2004 |
| | | | EP | 1432794 A2 | 30-06-2004 |
| | | | HU | 0500060 A2 | 28-04-2005 |
| | | | JP | 2005509413 T | 14-04-2005 |
| | | | MX | PA04002833 A | 15-07-2004 |
| | | | NO | 20041700 A | 26-04-2004 |
| | | | ZA | 200402262 A | 02-06-2004 |
| WO 2005075635 | A | 18-08-2005 | EP | 1713906 A2 | 25-10-2006 |
| | | | JP | 2007522805 T | 16-08-2007 |
| | | | US | 2007037746 A1 | 15-02-2007 |
| WO 2004110469 | A | 23-12-2004 | EP | 1646398 A2 | 19-04-2006 |
| | | | JP | 2006527216 T | 30-11-2006 |
| WO 2004111242 | A | 23-12-2004 | AU | 2004247799 A1 | 23-12-2004 |
| | | | BR | PI0411650 A | 08-08-2006 |
| | | | CA | 2529828 A1 | 23-12-2004 |
| | | | EP | 1644504 A1 | 12-04-2006 |
| | | | JP | 2006527982 T | 14-12-2006 |
| | | | KR | 20060022283 A | 09-03-2006 |
| | | | MX | PA05013769 A | 08-03-2006 |
| | | | US | 2005164932 A1 | 28-07-2005 |
| | | | US | 2006252128 A1 | 09-11-2006 |
| | | | US | 2006240526 A1 | 26-10-2006 |
| | | | US | 2006241041 A1 | 26-10-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4784950 A
- WO 0331464 A
- US 20040043446 A
- US 20040063911 A
- US 20040142856 A
- US 20040137557 A
- US 20040132640 A
- WO 0104287 A
- US 20030165996 A
- WO 0158935 A
- WO 0393465 A
- WO 0202764 A
- US 20030211094 A
- US 5580560 A
- WO 02077218 A
- WO 0238162 A
- WO 9920767 A
- US 6017882 A
- US 6747003 B
- US 20030100506 A
- WO 0066753 A
- US 20010018414 A
- US 2004220106 A
- US 200131005 B
- US 6762286 B
- US 6693075 B
- US 6806063 B
- US 20030096338 A
- WO 04029091 A
- WO 04083361 A
- WO 04111242 A
- WO 04108763 A
- WO 0183725 A
- WO 0222776 A
- WO 03027147 A
- WO 04029090 A
- JP 2001061479 B
- WO 2005111225 A
- EP 200421 A
- EP 06120000 A
- WO 2006013202 A
- WO 2006035057 A
- WO 2005014049 A
- WO 2005035553 A2
- WO 9520039 A
- WO 9838285 A
- WO 2005070468 A
- WO 2006013202 A2
- WO 2002077218 A
- EP 2006063310 W
- US 4456591 A, Thomas
- US 4837028 A
- US 4501728 A
- US 4975282 A
- DK 0200736 W
- WO 02062376 A
- WO 0185198 A
- DK 0200734 W
- DK 0200735 W
- DK 0200742 W
- DK 0200751 W
- DK 0200752 W
- WO 0185199 A
- DK 0200737 W
- DK 0200738 W
- DK 0200739 W
- DK 0200740 W
- DK 0200741 W
- WO 05075635 A
- EP 05108713 A

### Non-patent literature cited in the description

- **LENTING PJ et al.** *J Biol Chem,* 1999, vol. 274, 23734-23739
- **JURLANDER B et al.** *Thromb Hemost,* 2001, vol. 27, 373-383
- **WENGER RH et al.** *Biochem Biophys Res Commun,* 1988, vol. 156 (1), 389-95
- **BERNDT M C.** *Thromb Haemost,* 2001, vol. 86, 178-88
- **ANDREWS RK et al.** *Int J Biochem Cell Biol,* 2003, vol. 35, 1170-1174
- **WU G et al.** *Blood,* 1997, vol. 90, 2660-2669
- **LIAN J et al.** *Exp Cell Res,* 1999, vol. 252, 114-22
- **LINO et al.** *Arch. Biochem. Biophys,* 1998, vol. 352, 182-192
- **MOLLERUP et al.** *Biotechnol. Bioeng,* 1995, vol. 48, 501-505

- **KORNFELT et al.** *Arch. Biochem. Biophys,* 1999, vol. 363, 43-54
- **WEBER et al.** *Anal. Biochem,* 1995, vol. 225, 135
- **KLAUSEN et al.** *J. Chromatog,* 1995, vol. 718, 195
- **MORRIS et al.** Mass Spectrometry of Biological Materials. Marcel Dekker, 1990, 137-167
- **CONBOY et al.** *Biol. Mass Spectrom,* 1992, vol. 21, 397
- **HELLERQVIST.** *Meth. Enzymol,* 1990, vol. 193, 554
- **SUTTON et al.** *Anal. Biohcem,* 1994, vol. 318, 34
- **HARVEY et al.** *Organic Mass Spectrometry,* 1994, vol. 29, 752
- **HAGEN et al.** *Proc.Natl.Acad.Sci. USA,* 1986, vol. 83, 2412-2416
- **TAKEYA et al.** *J. Biol. Chem.,* 1988, vol. 263, 14868-14872
- **BROZE ; MAJERUS.** *J. Biol.Chem.,* 1980, vol. 255 (4), 1242-1247
- **HEDNER ; KISIEL.** *J. Clin.Invest,* 1983, vol. 71, 1836-1841
- **OSTERUD et al.** *Biochem,* 1972, vol. 11, 2853
- **HEDNER et al.** *J. Clin. Invest,* 1983, vol. 71, 1836
- **THIM L et al.** *Biochemistry,* 1988, vol. 27, 7785-7793
- **WAKABAYASHI et al.** *J. Biol. Chem.,* 1986, vol. 261, 11097
- **THIM et al.** *Biochem.,* 1988, vol. 27, 7785
- **SCOPES.** Protein Purification. Springer-Verlag, 1982
- Protein Purification. VCH Publishers, 1989
- **WEETERINGS C et al.** *Arterioscler Thromb Vasc Biol,* 2006, vol. 26 (3), 670-5
- **HUIZINGA EG et al.** *Science,* 2002, vol. 297 (5584), 1176-1179
- **LUO, S-Z et al.** *Blood,* 2007, vol. 109 (2), 603-609
- **LISMAN T et al.** *J Thromb Haemost,* 2005, vol. 3, 742-751
- **WARD C et al.** *Biochemistry,* 1996, vol. 35, 4929-4938
- **HOFFMEISTER KM et al.** *Science,* 2003, vol. 301, 1531-1534
- **KJALKE M et al.** *J Thromb Haemost,* 2007
- **KJALKE M et al.** *Thromb Haemost,* 1997, vol. 78, 1002-1008
- **H.M. BERMAN et al.** The Protein Data Bank. *Nucleic Acids Research,* 2000, vol. 28, 235-242
- **FRESKGAARD PO et al.** *Protein Sci,* 1996, vol. 5, 1531-40
- **SØRENSEN B ; INGERSLEV J.** *J Thromb Haemost,* 2004, vol. 2, 102-110
- **TRANHOLM M et al.** *Blood,* 2003, vol. 102, 3615-3620

SEQUENCE LISTING

<110>  Novo Nordisk A/S

<120>  FACTOR VII POLYPEPTIDES WITH INCREASED AFFINITY TO PLATELETS

<130>  7552-000-EP

<160>  1

<170>  PatentIn version 3.3

<210>  1
<211>  406
<212>  PRT
<213>  Homo sapiens


<220>
<221>  MOD_RES
<222>  (6)..(6)
<223>  GAMMA-CARBOXYGLUTAMIC ACID

<220>
<221>  MOD_RES
<222>  (7)..(7)
<223>  GAMMA-CARBOXYGLUTAMIC ACID

<220>
<221>  MOD_RES
<222>  (14)..(14)
<223>  GAMMA-CARBOXYGLUTAMIC ACID

<220>
<221>  MOD_RES
<222>  (16)..(16)
<223>  GAMMA-CARBOXYGLUTAMIC ACID

<220>
<221>  MOD_RES
<222>  (19)..(19)
<223>  GAMMA-CARBOXYGLUTAMIC ACID

<220>
<221>  MOD_RES
<222>  (20)..(20)
<223>  GAMMA-CARBOXYGLUTAMIC ACID

<220>
<221>  MOD_RES
<222>  (25)..(25)
<223>  GAMMA-CARBOXYGLUTAMIC ACID

<220>
<221>  MOD_RES
<222>  (26)..(26)
<223>  GAMMA-CARBOXYGLUTAMIC ACID

EP 1 952 822 A1

```
<220>
<221>   MOD_RES
<222>   (29)..(29)
<223>   GAMMA-CARBOXYGLUTAMIC ACID

<220>
<221>   MOD_RES
<222>   (35)..(35)
<223>   GAMMA-CARBOXYGLUTAMIC ACID

<400>  1
```

```
Ala Asn Ala Phe Leu Glu Glu Leu Arg Pro Gly Ser Leu Glu Arg Glu
1               5                   10                  15


Cys Lys Glu Glu Gln Cys Ser Phe Glu Glu Ala Arg Glu Ile Phe Lys
            20                  25                  30


Asp Ala Glu Arg Thr Lys Leu Phe Trp Ile Ser Tyr Ser Asp Gly Asp
        35                  40                  45


Gln Cys Ala Ser Ser Pro Cys Gln Asn Gly Gly Ser Cys Lys Asp Gln
        50                  55                  60


Leu Gln Ser Tyr Ile Cys Phe Cys Leu Pro Ala Phe Glu Gly Arg Asn
65                  70                  75                  80


Cys Glu Thr His Lys Asp Asp Gln Leu Ile Cys Val Asn Glu Asn Gly
                85                  90                  95


Gly Cys Glu Gln Tyr Cys Ser Asp His Thr Gly Thr Lys Arg Ser Cys
            100                 105                 110


Arg Cys His Glu Gly Tyr Ser Leu Leu Ala Asp Gly Val Ser Cys Thr
        115                 120                 125


Pro Thr Val Glu Tyr Pro Cys Gly Lys Ile Pro Ile Leu Glu Lys Arg
        130                 135                 140


Asn Ala Ser Lys Pro Gln Gly Arg Ile Val Gly Gly Lys Val Cys Pro
145                 150                 155                 160


Lys Gly Glu Cys Pro Trp Gln Val Leu Leu Leu Val Asn Gly Ala Gln
                165                 170                 175
```

22

Leu Cys Gly Gly Thr Leu Ile Asn Thr Ile Trp Val Val Ser Ala Ala
                180                 185                 190

His Cys Phe Asp Lys Ile Lys Asn Trp Arg Asn Leu Ile Ala Val Leu
                195                 200                 205

Gly Glu His Asp Leu Ser Glu His Asp Gly Asp Glu Gln Ser Arg Arg
    210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr
                260                 265                 270

Leu Ala Phe Val Arg Phe Ser Leu Val Ser Gly Trp Gly Gln Leu Leu
                275                 280                 285

Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320

Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
                340                 345                 350

Arg Gly Thr Trp Tyr Leu Thr Gly Ile Val Ser Trp Gly Gln Gly Cys
                355                 360                 365

Ala Thr Val Gly His Phe Gly Val Tyr Thr Arg Val Ser Gln Tyr Ile
                370                 375                 380

Glu Trp Leu Gln Lys Leu Met Arg Ser Glu Pro Arg Pro Gly Val Leu
385                 390                 395                 400

Leu Arg Ala Pro Phe Pro

405